# EUROPEAN PATENT APPLICATION

(11) **EP 1 238 973 A1**
(43) Date of publication of application: **11.09.2002**
(21) Application number: 00981686.9
(22) Date of filing: 13.12.2000
(51) Int. Cl.: C07D 209/88, C07D 307/91, C07D 333/76, A61K 31/343, A61K 31/381, A61K 31/403, A61P 3/04, A61P 3/06, A61P 3/10

(54) **NOVEL SUBSTITUTED TRICYCLIC COMPOUNDS**

(30) Priority: 16.12.1999 JP 35691499
(71) Applicant: Asahi Kasei Kabushiki Kaisha, Osaka-shi, Osaka 530-8205 (JP)
(72) Inventor: MIYOSHI, Shiro, Fuji-shi, Shizuoka 416-0946 (JP); OGAWA, Kohei, Mishima-shi, Shizuoka 411-0802 (JP)
(74) Representative: Forstmeyer, Dietmar, Dr. rer. nat., Dipl.-Chem.
(86) International application number: JP0008816
(87) International publication number: WO01044187

(57) **Abstract**

Compounds of the general formula (I) are useful in the treatment and prevention of β3-related diseases including diabetes, obesity and hyperlipidemia wherein R¹ is hydrogen, halogen, or hydroxyl; R² is lower alkyl or benzyl; R³ is OR, halogen, trifluoromethyl, lower alkyl, lower acyl, NR⁴R^{4'}, nitro, or cyano; R is hydrogen, lower alkyl, benzyl group, or optionally substituted lower acyl; R⁴ and R^{4'} are each independently hydrogen, lower alkyl, lower acyl, benzyl, or SO₂R⁵; R⁵ is lower alkyl or benzyl; W is oxygen, a secondary nitrogen atom (NH), or sulfur; and * represents an asymmetric carbon atom.

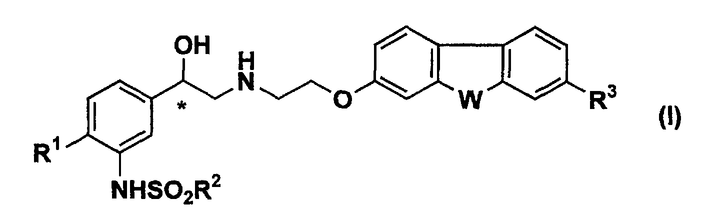

## Description

### TECHNICAL FIELD

This invention relates to novel substituted tricyclic compounds and pharmaceutical compositions containing the substituted tricyclic compounds.

### BACKGROUND OF THE INVENTION

In the past, β-adrenoreceptors were classified into two classes, β1-adrenoreceptor and β2-adrenoreceptor, and it was recognized that stimulation of β1 induces an increase in the heart rate and stimulation of β2 induces a relaxation of the smooth muscle tissue, thereby resulting in lowering the blood pressure. Arch, et al., clarified the presence of the third receptor by finding a compound which has very weak β1 and β2 activities and promotes the lipolysis of fatty cells (Nature, 309, p. 163 (1984)). Then, the primary structure of the third receptor was elucidated (Emorine, et al., Science, 245, p. 1118 (1989)), and it was named as β3. Recently, compounds having β3-agonist activity were shown to be useful as a medicine for treating and preventing diabetes, obesity, hyperlipidemia, digestive diseases and depression (Int. J. Obesity, 8 (Suppl. 1), p. 93 (1984); Nature, 309, p. 163 (1984); USP 5,120,766; Brit. J. Pharmacol., 103, p. 1351 (1991); Eur. J. Pharmacol., 219, p. 193 (1992)).

So far, the following compounds have been exemplified as compounds relating to β3:
the compound (BRL 37344) having the following structural formula described in EP 023385 and Drugs of the future, 16, p. 797 (1991): 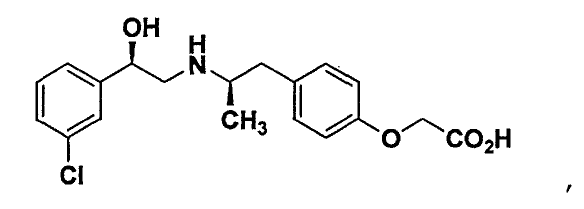
the compound (CL 316,243) having the following structural formula described in EP 0455006 and J. Med. Chem., 35, p 3081 (1992): 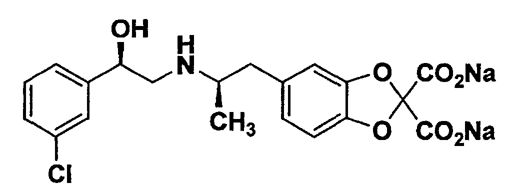 and
   the compound having the following structural formula described in WO 94/29290:

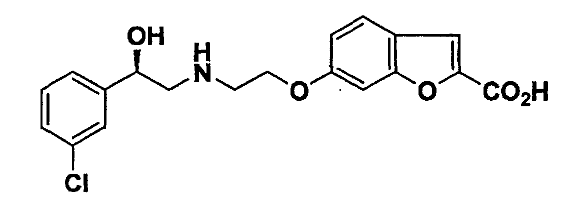

Further, EP 0659737 discloses a variety of compounds and specifically describes as an example in Example 1 in the specification the compound having the following structural formula: 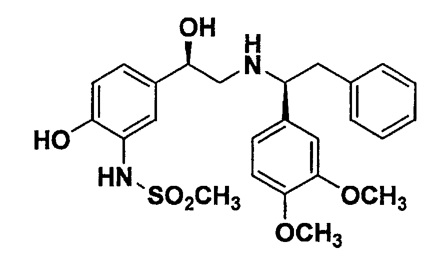 However, the chemical structures of the above compounds are clearly distinct from those of the claimed compounds of the present invention.

In addition, the compound described in EP 171702 and having the following structural formula: 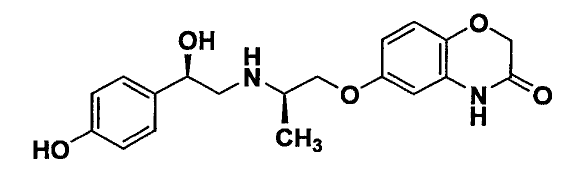 has been known as having heart rate-increasing activity, myocardial contraction enhancement and antiobestic activity. However, this compound acts on the heart and is different from the compound of the present invention in the chemical structure and in that the former strongly acts on the heart.

Further, the compound described in JP-A-55-53262 and JP-A-58-41860 and having the following structural formula: 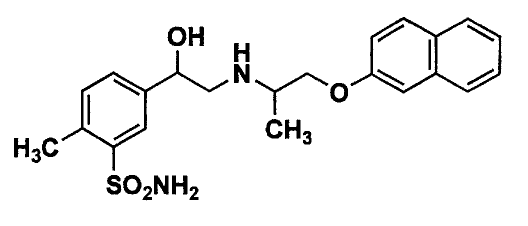 is known as having α,β-blocking activity, namely the effect of lowering blood pressure ,; and the compound described in DE 2651572 and having the following structural formula: 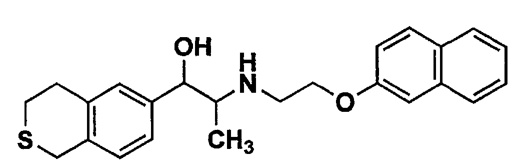 is known as having vasodilator action,. However, these compounds are different from the compounds of the present invention in their chemical structures and intended uses.

### DISCLOSURE OF THE INVENTION

There has been a need for a novel and useful medicine for treating and preventing β3-associated diseases, such as diabetes, obesity and hyperlipidemia.

In order to solve the above problems, the present inventors prepared a variety of compounds and investigated their activities. As a result, the present inventors have found that a novel tricyclic compound of the general formula (I) as set forth below has β3-agonist activity and has hypoglycemic action and lipolytic action, and then completed the present invention.

That is, the present invention is a compound of the general formula (I): 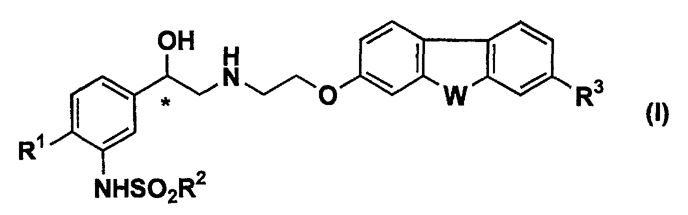 or a salt thereof,
wherein
R¹ represents a hydrogen atom, a halogen atom, or a hydroxyl group;
R² represents a lower alkyl group or a benzyl group;
R³ represents OR, a halogen atom, a trifluoromethyl group, a lower alkyl group, a lower acyl group, NR⁴R^{4'}, a nitro group, or a cyano group;
R represents a hydrogen atom, a lower alkyl group, a benzyl group, or an optionally substituted lower acyl group;
R⁴ and R^{4'} may be the same or different and represent a hydrogen atom, a lower alkyl group, a lower acyl group, a benzyl group, or SO₂R⁵;
R⁵ represents a lower alkyl group or a benzyl group;
W represents an oxygen atom, a secondary nitrogen atom (NH), or a sulfur atom; and
* represents an asymmetric carbon atom.

The disclosures in the text of specification of Japanese Patent Application No. 11-356914, from which the present application claims the priority right, is incorporated herein.

### DESCRIPTION OF PREFERRED EMBODIMENTS OF THE INVENTION

As used herein, "halogen atom" may be fluorine, chlorine, bromine or iodine atom with fluorine, chlorine and bromine atoms being preferred. In addition, "lower alkyl group" means a straight or branched saturated hydrocarbon containing 1 to 4 carbon atoms and includes methyl, ethyl, n-propyl, i-propyl, n-butyl, i-butyl, sec-butyl and tert-butyl groups. Further, "lower acyl group" means a straight or branched acyl group containing 1 to 6 carbon atoms and includes formyl, acetyl, propionyl, butyryl, isobutyryl, valeryl, isovaleryl, pivaloyl and hexanoyl groups.

R¹ is a hydrogen atom, a halogen atom or a hydroxyl group, and preferred examples thereof include hydrogen, fluorine, chlorine and bromine atoms and hydroxyl group.

R² is a lower alkyl group or a benzyl group, and specific examples thereof include methyl, ethyl, benzyl, n-propyl, i-propyl, n-butyl, i-butyl, sec-butyl and tert-butyl groups, with methyl and benzyl groups being particularly preferred.

R³ is OR, a halogen atom, a trifluoromethyl group, a lower alkyl group, a lower acyl group, NR⁴R^{4'}, a nitro group or a cyano group wherein R represents a hydrogen atom, a lower alkyl group, a benzyl group, or an optionally substituted lower acyl group; and R⁴ and R^{4'} may be the same or different and represent a hydrogen atom, a lower alkyl group, a lower acyl group, a benzyl group or SO₂R⁵, and R⁵ represents a lower alkyl group or a benzyl group. Preferred examples of R³ include OR and NR⁴R^{4'}. Preferred examples of R include a hydrogen atom, a lower alkyl group and an optionally substituted lower acyl group. More preferred examples of R⁴ and R^{4'} include a hydrogen atom, a lower acyl group and SO₂R⁵.

W represents an oxygen atom, a secondary nitrogen atom (NH), or a sulfur atom, with secondary nitrogen atom being preferred. Substituent of the optionally substituted lower acyl group is not specifically limited so long as it may be a substituent for a lower alkyl commonly included in a commercially available reagent. Preferred examples of such substituent include an amino group optionally substituted with a lower alkyl group, a hydroxyl group, a lower alkoxy group, or the like.

The term "leaving group" mentioned below means a removable group such as chlorine, bromine or iodine atom, or a sulfonic acid ester such as mesyl or tosyl group.

In the general formula (I) set forth above, * is an asymmetric carbon atom, and the compound of the general formula (I) can be in the form of any of two enantiomers, R-enantiomer and S-enantiomer. Not only optically pure isomers, but also mixtures of the two isomers with any mixing ratio are encompassed in the present invention. From the viewpoint of the expression of pharmacological activity, a preferred configuration of the asymmetric carbon * in the ethanolamino chain is the absolute configuration R. With respect to the asymmetric carbon * of N-[3-[2-[2-(7-hydroxy-9H-carbazol-2-yloxy)ethylamino]-1-hydroxyethyl] phenyl]methanesulfonamide, R-hydroxy structure is a particularly preferred example.

According to the present invention, a variety of combinations of the substituents can form some markedly preferred classes of the claimed compounds. R¹, R², R³, R⁴, R^{4'}, R⁵, R, W and * are as defined above, unless otherwise specified.

In the compounds of the present invention represented by the general formula (I) or salts thereof, compound having a combination of each of the substituents in which R¹ represents a hydrogen atom, a halogen atom or a hydroxyl group; R³ represents OR, a halogen atom, a trifluoromethyl group, a lower alkyl group, a lower acyl group, NR⁴R^{4'}, a nitro group or a cyano group; R represents a hydrogen atom, a lower alkyl group, a benzyl group or an optionally substituted lower acyl group; R⁴ and R^{4'} may be the same or different and represent a hydrogen atom, a lower alkyl group, a lower acyl group, a benzyl group or SO₂R⁵; and R⁵ represents a lower alkyl group or a benzyl group, are cited as preferred example.

In the present invention, compounds of the general formula (I) or salts thereof having a combination of each of the substituents, in which R¹ represents a hydrogen atom, a halogen atom or a hydroxyl group; R³ represents OR, a halogen atom, a trifluoromethyl group, a lower alkyl group, NR⁴R^{4'}, a nitro group or a cyano group; R represents a hydrogen atom, a lower alkyl group or a benzyl group; and R⁴ and R^{4'} may be the same or different and represent a hydrogen atom, a lower alkyl group or a benzyl group, are also cited as preferred examples.

In the present invention, compounds of the general formula (I) or salts thereof having a combination of each of substituents in which R¹ represents a hydrogen atom, a halogen atom or a hydroxyl group; and R³ represents OR, a halogen atom, a trifluoromethyl group, a lower alkyl group, NR⁴R^{4'} or a cyano group, are also cited as preferred examples.

In the present invention, compounds of the general formula (I) or salts thereof having a combination of each of substituents in which R¹ represents a hydrogen atom, a fluorine atom, a chlorine atom, a bromine atom or a hydroxyl group; R³ represents OR, a fluorine atom, a chlorine atom, a bromine atom, a trifluoromethyl group, a lower alkyl group or NR⁴R^{4'}; and R represents a lower alkyl group or a benzyl group, are also cited as preferred examples.

In the present invention, compounds of the general formula (I) or salts thereof having a combination of each of substituents in which R¹ represents a hydrogen atom, a fluorine atom, a chlorine atom, a bromine atom or a hydroxyl group; R³ represents OR; and R represents a hydrogen atom, a lower alkyl group, a benzyl group or an optionally substituted lower acyl group, are also cited as preferred examples.

In the present invention, compounds of the general formula (I) or salts thereof having a combination of each of substituents in which R¹ represents a hydrogen atom, a fluorine atom, a chlorine atom, a bromine atom or a hydroxyl group; R³ represents NR⁴R^{4'}; and R⁴ and R^{4'} may be the same or different and represent a hydrogen atom, a lower alkyl group, a lower acyl group, a benzyl group or SO₂R⁵, are also cited as preferred examples.

In the present invention, compounds of the general formula (I) or salts thereof having a combination of each of substituents in which R¹ represents a hydrogen atom, a fluorine atom, a chlorine atom, a bromine atom or a hydroxyl group; and R³ represents a hydroxyl group, are also cited as preferred examples.

In addition, the following compounds may be mentioned as specific compounds of the general formula (I) of the present invention.
(R)-N-[3-[2-[2-(7-hydroxy-9H-carbazol-2-yloxy)ethylamino]-1-hydroxyethyl]phenyl]methanesulfonamide;
(S)-N-[3-[2-[2-(7-hydroxy-9H-carbazol-2-yloxy)ethylamino]-1-hydroxyethyl]phenyl]methanesulfonamide;
N-[3-[2-[2-(7-hydroxy-9H-carbazol-2-yloxy)ethylamino]-1-hydroxyethyl]phenyl]methanesulfonamide;
(R)-N-[3-[2-[2-(7-chloro-9H-carbazol-2-yloxy)ethylamino]-1-hydroxyethyl]phenyl]methanesulfonamide;
(S)-N- [3-[2-[2-(7-chloro-9H-carbazol-2-yloxy)ethylamino]-1-hydroxyethyl]phenyl]methanesulfonamide;
N-[3-[2-[2-(7-chloro-9H-carbazol-2-yloxy)ethylamino]-1-hydroxyethyl]phenyl]methanesulfonamide;
(R)-N-[3-[2-[2-(7-bromo-9H-carbazol-2-yloxy)ethylamino]-1-hydroxyethyl]phenyl]methanesulfonamide;
(S)-N-[3-[2-[2-(7-bromo-9H-carbazol-2-yloxy)ethylamino]-1-hydroxyethyl]phenyl]methanesulfonamide;
N-[3-[2-[2-(7-bromo-9H-carbazol-2-yloxy)ethylamino]-1-hydroxyethyl]phenyl]methanesulfonamide;
(R)-N-[3-[2-[2-(7-methoxy-9H-carbazol-2-yloxy)ethylamino]-1-hydroxyethyl]phenyl]methanesulfonamide;
(S)-N- [3-[2-[2-(7-methoxy-9H-carbazol-2-yloxy)ethylamino]-1-hydroxyethyl]phenyl]methanesulfonamide;
N-[3-[2-[2-(7-methoxy-9H-carbazol-2-yloxy)ethylamino]-1-hydroxyethyl]phenyl]methanesulfonamide;
(R)-N-[3-[2-[2-(7-trifluoromethyl-9H-carbazol-2-yloxy)ethylamino]-1-hydroxyethyl]phenyl]methanesulfonamide;
(S)-N-[3-[2-[2-(7-trifluoromethyl-9H-carbazol-2-yloxy)ethylamino]-1-hydroxyethyl]phenyl]methanesulfonamide;
N-[3-[2-[2-(7-trifluoromethyl-9H-carbazol-2-yloxy)ethylamino]-1-hydroxyethyl]phenyl]methanesulfonamide;
(R)-N-[3-[2-[2-(7-methyl-9H-carbazol-2-yloxy)ethylamino]-1-hydroxyethyl]phenyl]methanesulfonamide;
(S)-N-[3-[2-[2-(7-methyl-9H-carbazol-2-yloxy)ethylamino]-1-hydroxyethyl]phenyl] methanesulfonamide;
N-[3-[2-[2-(7-methyl-9H-carbazol-2-yloxy)ethylamino]-1-hydroxyethyl]phenyl]methanesulfonamide;
(R)-N-[3-[2-[2-(7-cyano-9H-carbazol-2-yloxy)ethylamino]-1-hydroxyethyl]phenyl]methanesulfonamide;
(S)-N-[3-[2-[2-(7-cyano-9H-carbazol-2-yloxy)ethylamino]-1-hydroxyethyl]phenyl]methanesulfonamide;
N-[3-[2-[2-(7-cyano-9H-carbazol-2-yloxy)ethylamino]-1-hydroxyethyl]phenyl]methanesulfonamide;
(R)-N-[5-[2-[2-(7-hydroxy-9H-carbazol-2-yloxy)ethylamino]-1-hydroxyethyl]-2-chlorophenyl]methanesulfonamide;
(S)-N-[5-[2-[2-(7-hydroxy-9H-carbazol-2-yloxy)ethylamino]-1-hydroxyethyl]-2-chlorophenyl]methanesulfonamide;
N-[5-[2-[2-(7-hydroxy-9H-carbazol-2-yloxy)ethylamino]-1-hydroxyethyl]-2-chlorophenyl]methanesulfonamide;
(R)-N-[5-[2-[2-(7-chloro-9H-carbazol-2-yloxy)ethylamino]-1-hydroxyethyl]-2-chlorophenyl]methanesulfonamide;
(S)-N-[5-[2-[2-(7-chloro-9H-carbazol-2-yloxy)ethylamino]-1-hydroxyethyl]-2-chlorophenyl]methanesulfonamide;
N-[5-[2-[2-(7-chloro-9H-carbazol-2-yloxy)ethylamino]-1-hydroxyethyl]-2-chlorophenyl]methanesulfonamide;
(R)-N-[5-[2-[2-(7-bromo-9H-carbazol-2-yloxy)ethylamino]-1-hydroxyethyl]-2-chlorophenyl]methanesulfonamide;
(S)-N- [5-[2-[2-(7-bromo-9H-carbazol-2-yloxy)ethylamino]-1-hydroxyethyl]-2-chlorophenyl]methanesulfonamide;
N-[5-[2-[2-(7-bromo-9H-carbazol-2-yloxy)ethylamino]-1-hydroxyethyl]-2-chlorophenyl]methanesulfonamide;
(R)-N-[5-[2-[2-(7-methoxy-9H-carbazol-2-yloxy)ethylamino]-1-hydroxyethyl]-2-chlorophenyl]methanesulfonamide;
(S)-N-[5-[2-[2-(7-methoxy-9H-carbazol-2-yloxy)ethylamino]-1-hydroxyethyl]-2-chlorophenyl]methanesulfonamide;
N-[5-[2-[2-(7-methoxy-9H-carbazol-2-yloxy)ethylamino]-1-hydroxyethyl]-2-chlorophenyl]methanesulfonamide;
(R)-N-[5-[2-[2-(7-trifluoromethyl-9H-carbazol-2-yloxy)ethylamino]-1-hydroxyethyl]-2-chlorophenyl]methanesulfonamide;
(S)-N-[5-[2-[2-(7-trifluoromethyl-9H-carbazol-2-yloxy)ethylamino]-1-hydroxyethyl]-2-chlorophenyl]methanesulfonamide;
N-[5-[2-[2-(7-trifluoromethyl-9H-carbazol-2-yloxy)ethylamino]-1-hydroxyethyl]-2-chlorophenyl]methanesulfonamide;
(R)-N-[5-[2-[2-(7-methyl-9H-carbazol-2-yloxy)ethylamino]-1-hydroxyethyl]-2-chlorophenyl]methanesulfonamide;
(S)-N- [5-[2-[2-(7-methyl-9H-carbazol-2-yloxy)ethylamino]-1-hydroxyethyl]-2-chlorophenyl]methanesulfonamide;
N-[5-[2-[2-(7-methyl-9H-carbazol-2-yloxy)ethylamino]-1-hydroxyethyl]-2-chlorophenyl]methanesulfonamide;
(R)-N-[5-[2-[2-(7-cyano-9H-carbazol-2-yloxy)ethylamino]-1-hydroxyethyl]-2-chlorophenyl]methanesulfonamide;
(S)-N-[5-[2-[2-(7-cyano-9H-carbazol-2-yloxy)ethylamino]-1-hydroxyethyl]-2-chlorophenyl]methanesulfonamide;
N-[5-[2-[2-(7-cyano-9H-carbazol-2-yloxy)ethylamino]-1-hydroxyethyl]-2-chlorophenyl]methanesulfonamide;
(R)-N-[5-[2-[2-(7-hydroxy-9H-carbazol-2-yloxy)ethylamino]-1-hydroxyethyl]-2-bromophenyl]methanesulfonamide;
(S)-N- [5-[2-[2-(7-hydroxy-9H-carbazol-2-yloxy)ethylamino]-1-hydroxyethyl]-2-bromophenyl]methanesulfonamide;
N-[5- [2-[2-(7-hydroxy-9H-carbazol-2-yloxy)ethylamino]-1-hydroxyethyl]-2-bromophenyl]methanesulfonamide;
(R)-N-[5-[2-[2-(7-chloro-9H-carbazol-2-yloxy)ethylamino]-1-hydroxyethyl]-2-bromophenyl]methanesulfonamide;
(S)-N-[5-[2-[2-(7-chloro-9H-carbazol-2-yloxy)ethylamino]-1-hydroxyethyl]-2-bromophenyl]methanesulfonamide;
N-[5-[2-[2-(7-chloro-9H-carbazol-2-yloxy)ethylamino]-1-hydroxyethyl]-2-bromophenyl]methanesulfonamide;
(R)-N-[5-[2-[2-(7-bromo-9H-carbazol-2-yloxy)ethylamino]-1-hydroxyethyl]-2-bromophenyl]methanesulfonamide;
(S)-N-[5-[2-[2-(7-bromo-9H-carbazol-2-yloxy)ethylamino]-1-hydroxyethyl]-2-bromophenyl]methanesulfonamide;
N- [5- [2-[2-(7-bromo-9H-carbazol-2-yloxy)ethylamino]-1-hydroxyethyl]-2-bromophenyl]methanesulfonamide;
(R) -N- [5-[2-[2-(7-methoxy-9H-carbazol-2-yloxy)ethylamino]-1-hydroxyethyl]-2-bromophenyl]methanesulfonamide;
(S)-N-[5-[2-[2-(7-methoxy-9H-carbazol-2-yloxy)ethylamino]-1-hydroxyethyl]-2-bromophenyl]methanesulfonamide;
N-[5-[2-[2-(7-methoxy-9H-carbazol-2-yloxy)ethylamino]-1-hydroxyethyl]-2-bromophenyl]methanesulfonamide;
(R)-N-[5-[2-[2-(7-trifluoromethyl-9H-carbazol-2-yloxy)ethylamino]-1-hydroxyethyl]-2-bromophenyl]methanesulfonamide;
(S)-N-[5-[2-[2-(7-trifluoromethyl-9H-carbazol-2-yloxy)ethylamino]-1-hydroxyethyl]-2-bromophenyl]methanesulfonamide;
N-[5-[2-[2-(7-trifluoromethyl-9H-carbazol-2-yloxy)ethylamino]-1-hydroxyethyl]-2-bromophenyl]methanesulfonamide;
(R)-N-[5-[2-[2-(7-methyl-9H-carbazol-2-yloxy)ethylamino]-1-hydroxyethyl]-2-bromophenyl]methanesulfonamide;
(S)-N-[5-[2-[2-(7-methyl-9H-carbazol-2-yloxy)ethylamino]-1-hydroxyethyl]-2-bromophenyl]methanesulfonamide;
N-[5-[2-[2-(7-methyl-9H-carbazol-2-yloxy)ethylamino]-1-hydroxyethyl]-2-bromophenyl]methanesulfonamide;
(R)-N-[5-[2-[2-(7-cyano-9H-carbazol-2-yloxy)ethylamino]-1-hydroxyethyl]-2-bromophenyl]methanesulfonamide;
(S)-N-[5-[2-[2-(7-cyano-9H-carbazol-2-yloxy)ethylamino]-1-hydroxyethyl]-2-bromophenyl]methanesulfonamide;
N-[5-[2-[2-(7-cyano-9H-carbazol-2-yloxy)ethylamino]-1-hydroxyethyl]-2-bromophenyl]methanesulfonamide;
(R)-N- [5-[2-[2-(7-hydroxy-9H-carbazol-2-yloxy)ethylamino]-1-hydroxyethyl]-2-hydroxyphenyl]methanesulfonamide;
(S)-N-[5-[2-[2-(7-hydroxy-9H-carbazol-2-yloxy)ethylamino]-1-hydroxyethyl]-2-hydroxyphenyl]methanesulfonamide;
N-[5-[2-[2-(7-hydroxy-9H-carbazol-2-yloxy)ethylamino]-1-hydroxyethyl]-2-hydroxyphenyl]methanesulfonamide;
(R)-N-[5-[2-[2-(7-chloro-9H-carbazol-2-yloxy)ethylamino]-1-hydroxyethyl]-2-hydroxyphenyl]methanesulfonamide;
(S)-N-[5-[2-[2-(7-chloro-9H-carbazol-2-yloxy)ethylamino]-1-hydroxyethyl]-2-hydroxyphenyl]methanesulfonamide;
N-[5-[2-[2-(7-chloro-9H-carbazol-2-yloxy)ethylamino]-1-hydroxyethyl]-2-hydroxyphenyl]methanesulfonamide;
(R)-N-[5-[2-[2-(7-bromo-9H-carbazol-2-yloxy)ethylamino]-1-hydroxyethyl]-2-hydroxyphenyl]methanesulfonamide;
(S)-N-[5-[2-[2-(7-bromo-9H-carbazol-2-yloxy)ethylamino]-1-hydroxyethyl]-2-hydroxyphenyl]methanesulfonamide;
N-[5-[2-[2-(7-bromo-9H-carbazol-2-yloxy)ethylamino]-1-hydroxyethyl]-2-hydroxyphenyl]methanesulfonamide;
(R)-N-[5-[2-[2-(7-methoxy-9H-carbazol-2-yloxy)ethylamino]-1-hydroxyethyl]-2-hydroxyphenyl]methanesulfonamide;
(S)-N-[5-[2-[2-(7-methoxy-9H-carbazol-2-yloxy)ethylamino]-1-hydroxyethyl]-2-hydroxyphenyl]methanesulfonamide;
N-[5-[2-[2-(7-methoxy-9H-carbazol-2-yloxy)ethylamino]-1-hydroxyethyl]-2-hydroxyphenyl]methanesulfonamide;
(R)-N-[5-[2-[2-(7-trifluoromethyl-9H-carbazol-2-yloxy)ethylamino]-1-hydroxyethyl]-2-hydroxyphenyl]methanesulfonamide;
(S)-N-[5-[2-[2-(7-trifluoromethyl-9H-carbazol-2-yloxy)ethylamino]-1-hydroxyethyl]-2-hydroxyphenyl]methanesulfonamide;
N-[5-[2-[2-(7-trifluoromethyl-9H-carbazol-2-yloxy)ethylamino]-1-hydroxyethyl]-2-hydroxyphenyl]methanesulfonamide;
(R)-N-[5-[2-[2-(7-methyl-9H-carbazol-2-yloxy)ethylamino]-1-hydroxyethyl]-2-hydroxyphenyl]methanesulfonamide;
(S)-N-[5-[2-[2-(7-methyl-9H-carbazol-2-yloxy)ethylamino]-1-hydroxyethyl]-2-hydroxyphenyl]methanesulfonamide;
N-[5-[2-[2-(7-methyl-9H-carbazol-2-yloxy)ethylamino]-1-hydroxyethyl]-2-hydroxyphenyl]methanesulfonamide;
(R)-N-[5-[2-[2-(7-cyano-9H-carbazol-2-yloxy)ethylamino]-1-hydroxyethyl]-2-hydroxyphenyl]methanesulfonamide;
(S)-N-[5-[2-[2-(7-cyano-9H-carbazol-2-yloxy)ethylamino]-1-hydroxyethyl]-2-hydroxyphenyl]methanesulfonamide;
N-[5-[2-[2-(7-cyano-9H-carbazol-2-yloxy)ethylamino]-1-hydroxyethyl]-2-hydroxyphenyl]methanesulfonamide;
(R)-N-[5-[2-[2-(7-amino-9H-carbazol-2-yloxy)ethylamino]-1-hydroxyethyl]phenyl]methanesulfonamide;
(R)-N-[5-[2-[2-(7-amino-9H-carbazol-2-yloxy)ethylamino]-1-hydroxyethyl]-2-chlorophenyl]methanesulfonamide;
(R)-N-[5-[2-[2-(7-acetylamino-9H-carbazol-2-yloxy)ethylamino]-1-hydroxyethyl]phenyl]methanesulfonamide; and
(R)-N-[5-[2-[2-(7-acetylamino-9H-carbazol-2-yloxy)ethylamino]-1-hydroxyethyl]-2-chlorophenyl]methanesulfonamide.

Compounds of the general formula (I) can be prepared, for example, by the following processes.

### [Preparation Process A]

Preparation Process A is a process comprising reacting a compound of the general formula (II): 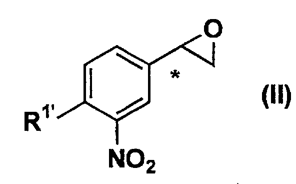 wherein R^{1'} represents a hydrogen atom, a halogen atom, or a protected hydroxyl group, and * represents an asymmetric carbon atom, with a compound of the general formula (III): 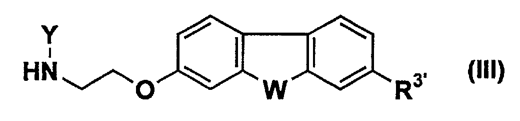 wherein W represents an oxygen atom, a secondary nitrogen atom (NH), or a sulfur atom; Y represents a hydrogen atom or an amine-protecting group; R^{3'} represents OR', a halogen atom, a trifluoromethyl group, a lower alkyl group, a lower acyl group, NR⁴R^{4'}, a nitro group, or a cyano group; R' represents a lower alkyl group, an optionally substituted lower acyl group, a benzyl group, or a hydroxyl-protecting group; R⁴ and R^{4'} may be the same or different and represent a hydrogen atom, a lower alkyl group, a lower acyl group, a benzyl group, an amine-protecting group, or SO₂R⁵; and R⁵ represents a lower alkyl group or a benzyl group, to give a compound of the general formula (IV): 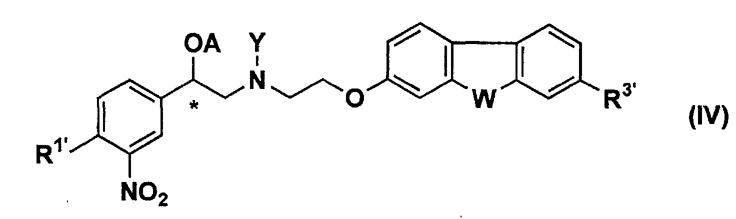 wherein A represents a hydrogen atom, and R^{1'}, R^{3'}, W, Y and * are each as defined above;
converting Y to an amine-protecting group when Y is a hydrogen atom;
reducing the compound of the general formula (IV) in which Y represents an amine-protecting group, to give a compound of the general formula (V): 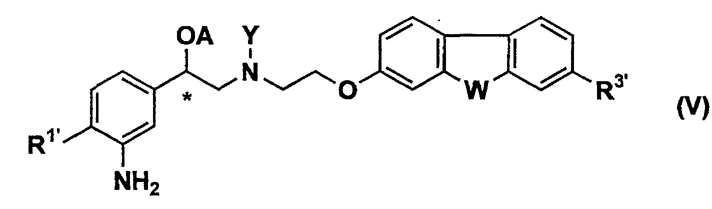 wherein Y represents an amine-protecting group, and A, R^{1'}, R^{3'} W and * are each as defined above;
reacting the compound of the general formula (V) with a compound of the general formula (VI):

**XSO**_{**2**}**R**^{**2**} **(VI)**

wherein R² represents a lower alkyl group or a benzyl group, and X represents a leaving group wherein the leaving group means a removable group such as chlorine, bromine or iodine atom, or a sulfonic acid ester such as mesyl or tosyl group, in the presence of an alkali, to give a compound of the general formula (VII): 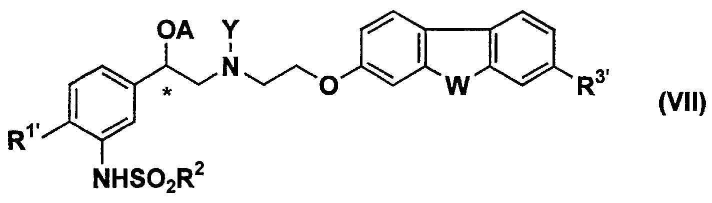 wherein A, R^{1'}, R², R^{3'}, W, Y and * are each as defined above; and
when at least one of R^{1'}, R^{3'} and Y comprises a protecting group, simultaneously or sequentially deprotecting it to give a compound of the general formula (I): 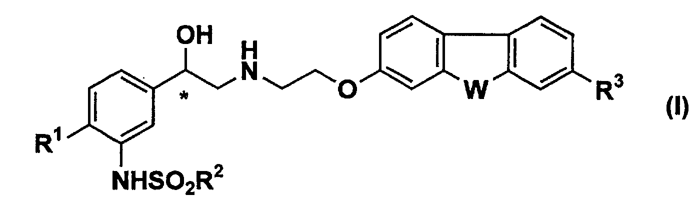 wherein R¹ represents a hydrogen atom, a halogen atom, or a hydroxyl group; R³ represents OR, a halogen atom, a trifluoromethyl group, a lower alkyl group, a lower acyl group, NR⁴R^{4'}, a nitro group, or a cyano group; R represents a hydrogen atom, a lower alkyl group, a benzyl group, or an optionally substituted lower acyl group; R⁴ and R^{4'} may be the same or different and represent a hydrogen atom, a lower alkyl group, a lower acyl group, a benzyl group, or SO₂R⁵; and R², R⁵, W and * are each as defined above.

When R^{1'} and/or R^{3'} comprises a hydroxyl-protecting group, the hydroxyl-protecting group is not limited as long as it is commonly used as a hydroxyl-protecting group. Preferred examples of easily and selectively removable hydroxyl-protecting group normally include a trialkylsilyl group, an alkoxyalkyl group , an acyl group and the like. These hydroxyl-protecting groups can be introduced and removed by a known method described in literatures accepted in the art (for example, T. W. Greene, P. G. M. Wuts, et al., Protective Groups in Organic Synthesis, Wiley-Interscience Publication)). For example, a tert-butyldimethylsilyl group (TBDMS) may be introduced into the alcohol by a treatment of the alcohol with a sililating agent such as tert-butyldimethylchlorosilane or tert-butyldimethylsilyl trifluoromethanesulfonate in the presence of an acid scavenger. The amount of the sililating agent to be added is normally about 1.0 to 1.5 mol for 1 mol of the alcohol. Normally, this reaction is preferably carried out in an inert medium. The inert medium may be dichloromethane, tetrahydrofuran, acetonitrile, pyridine and the like. N,N-dimethylformamide is an example of the preferred inert medium. The amount of the inert medium to be used may be about 1 to 5 mL for 1 g of the alcohol. The acid scavenger may be triethylamine, N,N-diisopropylethylamine, pyridine,N,N-dimethylaminopyridine and the like. The acid scavenger may be preferably imidazole. The amount of the acid scavenger to be added may be normally about 1 to 3 mol for 1 mol of the alcohol. Normally, this reaction is preferably carried out at a temperature of from about -20°C to about 80°C, particularly from about 0°C to room temperature, for example, for 1 to 5 hours.

A benzyloxymethyl group (BOM) may be introduced into the alcohol by a treatment of the alcohol with chloromethyl benzyl ether in the presence of an acid scavenger. The amount of chloromethyl benzyl ether to be added may be generally about 1.0 to 1.5 mol for 1 mol of the alcohol. Generally, this reaction is preferably carried out in an inert medium. The inert medium may be tetrahydrofuran, acetonitrile, N,N-dimethylformamide and the like. The inert medium may be preferably dichloromethane. The amount of the inert medium to be used may be about 1 to 5 mL for 1 g of the alcohol. The acid scavenger may be triethylamine, pyridine, N,N-dimethylaminopyridine and the like. An example of the preferred acid scavenger is N,N-diisopropylethylamine. The amount of the acid scavenger to be added may be normally about 1 to 3 mol for 1 mol of the alcohol. Generally, this reaction is preferably carried out at a temperature of from about -20°C to about 80°C, particularly from about 0°C to room temperature, for example, for 1 to 5 hours.

In addition, an acetyl group (Ac) may be introduced into the alcohol , for example, by a treatment of the alcohol with an acetylating agent such as acetic anhydride , acetyl chloride or the like in the presence of an acid scavenger. The amount of the acetylating agent to be added may be generally about 1 to 3 mol for 1 mol of the alcohol. Normally, this reaction is preferably carried out in an inert medium. The examples of the preferred inert medium are tetrahydrofuran, acetonitrile, dichloromethane, pyridine and the like. The amount of the inert medium to be used may be about 1 to 5 mL for 1 g of the alcohol. Examples of the preferred acid scavenger are triethylamine, N,N-diisopropylethylamine, pyridine, N,N-dimethylaminopyridine and the like. The amount of the acid scavenger to be added may be generally about 1 to 3 mol for 1 mol of the alcohol. Generally, this reaction is preferably carried out at a temperature of from about -20°C to about 80°C, particularly from about 0°C to room temperature, for example, for 1 to 5 hours.

In addition, when Y, R⁴ and/or R^{4'} comprises an amino-protecting group, the amino-protecting group may be, for example, an acyl group, an acyloxy group, or an easily removable aralkyl group. Examples of the easily removable aralkyl group include a benzyl group, a substituted benzyl group, a naphthylmethyl group, a substituted naphthylmethyl group and the like. A particularly preferred example is a benzyl group. The aralkyl group to be used may be an aralkyl group having 7 to 16 carbon atoms. Specific examples thereof include benzyl, phenethyl, 3-phenylpropyl, 4-phenylbutyl, (1-naphthyl)methyl, 2-(1-naphthyl)ethyl and 2-(2-naphthyl)ethyl groups. These aralkyl groups may have one or more suitable substituents, such as alkyl group, alkoxy group and halogen atom on suitable positions of the phenyl and naphthyl rings. These protecting groups may be introduced by a known method described in the abovementioned literatures accepted in the art.

A compound of the general formula (IV) is a novel substance and is characteristic as an important intermediate for synthesizing a compound of the general formula (I). A compound of the general formula (IV) is obtained by reacting a compound of the general formula (II) with a compound of the general formula (III) in a conventional medium, for example in an organic solvent such as dimethylsulfoxide, a linear or cyclic ether, dimethylformamide, dimethylacetamide, or an alcohol solvent, such as 2-butanol.

Though a compound of the general formula (II) and a compound of the general formula (III) are usually used in an equimolar amount, the latter is preferably used in an excess amount. The reaction temperature can be suitably selected and may be generally a temperature of from room temperature to the reflux temperature of the selected solvent. The reaction time can be suitably selected depending on the reaction conditions and the reaction may be normally completed when the yield is the highest. In addition, there is a report (Tetrahedron Letters, 27, p. 2451 (1986)) that the addition of trimethylsilylacetamide (TMSA), N,O-bis(trimethylsilyl)acetamide, hexamethyldisilazane (HMDS) or bis(trimethylsilyl)urea to the reaction can shorten the reaction time and improve the yield. This may be suitably applied to the present reaction.

In addition, a compound of the general formula (V) is also a novel substance and is characteristic as an important intermediate for synthesizing a compound of the general formula (I). A compound of the general formula (V) may be obtained by reducing the nitro group of a compound of the general formula (IV) to amine (aniline). When Y of the general formula (IV) is a hydrogen atom, Y is converted to an amine-protecting group prior to such a reduction reaction. The reduction reaction can be carried out by hydrogenating the compound in the presence of platinum oxide as a catalyst in a solvent such as methanol, or by reducing the compound using hydrochloric acid in the presence of iron powder or bivalent tin.

In addition, a compound of the general formula (VII) is also a novel substance and is characteristic as an important intermediate for synthesizing a compound of the general formula (I). A compound of the general formula (VII) may be obtained by sulfonating amine (aniline) of a compound of the general formula (V) with a compound of the general formula (VI) which provides various substituents as R², according to the method described in C. Kaiser, et al., J. Med. Chem., 17, p. 49 (1974). When R^{1'}, R^{3'} and/or Y comprise amine-protecting groups and/or hydroxyl-protecting groups, they are removed by the deprotecting method set forth below to give a compound of the general formula (I).

The above sulfonation reaction may be a reaction of a known or commercially available compound of the general formula (VI) with a compound of the general formula (V) in a solvent such as pyridine at a temperature of from ice cooling to room temperature. The deprotecting processes may be sequentially or simultaneously carried out. Preferably, the hydroxyl-protecting group in R^{1'} or R^{3'} may be first removed, followed by the removal of the amino-protecting groups in Y and R^{3'}. The deprotecting conditions are as follows. A benzyl group as the hydroxyl-protecting group in R^{1'} and R^{3'} is removed by a hydrogenolysis reaction with a catalyst such as palladium or nickel in a solvent such as methanol. Alternatively, a benzyl or methyl group as the hydroxyl-protecting group in R^{1'} and R^{3'} is removed by a treatment with a Lewis acid such as boron tribromide in a solvent such as methylene chloride. In addition, an acetyl group as the hydroxyl-protecting group in R^{1'} and R^{3'} is removed using known ester hydrolysis reaction conditions. A specific example may be a process comprising heating the compound in the presence of an alkali in an alcohol solvent at a temperature of from room temperature to the reflux temperature of the solvent. In addition, an triethylsilyl group as the hydroxyl-protecting group in R^{1'} and R^{3'} can be removed by treating the compound with acetic acid and 3 to 5 mol of tetrabutylammonium fluoride for 1 mol of the compound in tetrahydrofuran at room temperature for 0.5 to 5 hours. A benzyl group as the amino-protecting group in Y and R^{3'} can be removed by a hydrogenolysis reaction with a catalyst such as palladium or nickel in a solvent such as methanol. In addition, an acetyl group as the amine-protecting group in Y and R^{3'} can be removed by a treatment with hydrochloric acid in a solvent such as methanol at room temperature, or a heating treatment in the presence of an alkali in a solvent such as water or methanol.

A compound of the general formula (II) is a known substance. A racemic modification thereof can be obtained, for example, by oxidizing a known corresponding styrene compound with an oxidizing agent such as m-chloroperbenzoic acid in a solvent such as dichloromethane at a temperature of from about 0°C to room temperature.

Alternatively, a compound of the general formula (II) may be obtained by reducing a compound of the general formula (VIII): 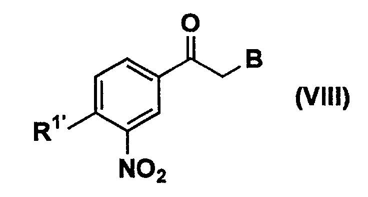 wherein R^{1'} is as defined above, and B represents a chlorine, bromine or iodine atom, according to the below mentioned method or the like to give a compound of the general formula (IX): 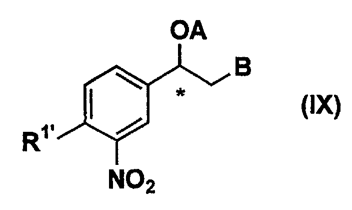 wherein R^{1'} and * are each as defined above, A represents a hydrogen atom, and B represents a chlorine, bromine or iodine atom;

when the compound having an iodine atom as the substituent B is to be obtained, replacing the chlorine or bromine atom with a iodine atom; and then epoxidizing the compound of the formula (IX) by an alkali treatment. That is, when the configuration * of the hydroxyl group of a compound of the general formula (IX) is racemic, a compound of the general formula (VIII) can be reduced with a reducing agent, such as borane.

In addition, if an optical isomer of either R-form or S-form with respect to * of the general formula (IX) is to be obtained, it can be obtained using a chiral auxiliary agent, such as a material represented by the general formula (X): 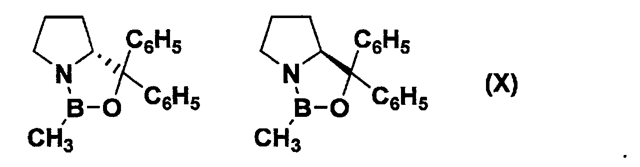 That is, it can be obtained by reducing a compound of the general formula (VIII) with borane in the presence of the above chiral auxiliary agent. The above reduction reaction is preferably carried out in a solvent, such as tetrahydrofuran. A process for the preparation of these chiral auxiliary agents and reactions thereof may be carried out in accordance with the teachings of E. J. Corey, et al., J. Org. Chem., 56, p. 442 (1991).

When the replacement of the chlorine or bromine atom with an iodine atom is needed after the reduction of a compound of the general formula (VIII) to a compound of the general formula (IX), there is exemplified a method of heating the reduced compound with an iodinating agent such as 3 to 10 mol of sodium iodide for 1 mol of the brominated form in a solvent such as acetone at the reflux temperature for 1 to 3 hours. Thereafter, the thus obtained compound is epoxidized in the presence of an alkali such as 1 to 2 equivalents of sodium hydroxide aqueous solution in a solvent such as methanol at a temperature of from about 0°C to room temperature to give a compound of the general formula (II). When a compound of the general formula (II) is obtained from a compound of the general formula (IX), the configuration with respect to the asymmetric carbon * is retained. That is, R-form generates R-form, and S-form generates S-form.

A compound of the general formula (VIII), which is a known compound, is commercially available or can be prepared according to the process described in, for example, A. A. Larsen, et al., J. Med. Chem., 10, p. 462 (1967), or C. Kaiser, et al., J. Med. Chem., 17, p. 49 (1974).

A compound of the general formula (III) is a novel substance and is characteristic as an important intermediate for the preparation of a compound of the general formula (I).

A compound of the general formula (III) is obtained by reacting a compound of the general formula (XI): 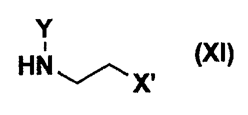 wherein Y represents an amine-protecting group, and X' represents a chlorine atom, a bromine atom or a hydroxyl group, with a compound of the general formula (XII): 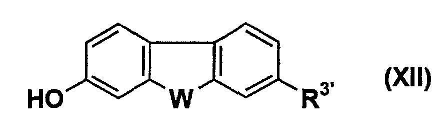 wherein W represents an oxygen atom, a secondary nitrogen atom (NH), or a sulfur atom; R^{3'} represents OR', a halogen atom, a trifluoromethyl group, a lower alkyl group, a lower acyl group, NR⁴R^{4'}, a nitro group, or a cyano group; R' represents a lower alkyl group, an optionally substituted lower acyl group, a benzyl group, or a hydroxyl-protecting group; R⁴ and R^{4'} may be the same or different and represent a hydrogen atom, a lower alkyl group, a lower acyl group, a benzyl group, an amine-protecting group, or SO₂R⁵; and R⁵ represents a lower alkyl group or benzyl group. Y, R⁴ or R^{4'} is an amine-protecting group, and is not limited as long as it is commonly used as an amine-protecting group. Examples of the amine-protecting group include a benzyl group, a benzyloxycarbonyl group, a substituted benzyloxycarbonyl group, tert-butoxycarbonyl group, a trifluoroacetyl group or the like, which is normally easily removable.

The reaction of a compound of the general formula (XI) with a compound of the general formula (XII) wherein X' represents a chlorine or bromine atom, is carried out , for example, in the presence of a base in an organic solvent at a temperature between room temperature and the reflux temperature of the selected solvent. Such solvents include dimethylformamide, dimethylacetamide, acetonitrile, diglyme and tetrahydrofuran. The base, such as potassium carbonate, sodium carbonate, sodium hydroxide, potassium hydroxide, triethylamine, pyridine, sodium hydride, sodium methoxide or the like is preferably used in an amount of 1 to 10 mol for 1 mol of a compound of the formula (XII).

When the reaction slowly proceeds, a compound of the general formula (III) in which Y represents an amine-protecting group may be prepared according to the process described in Bull. Chem. Soc. Jpn., 55, p. 2504 (1982) or an improved process thereof. An exemplified process comprises reacting a compound of the general formula (XII) with 2 to 5 mol of a compound of the general formula (XI) and 5 to 10 mol of 40% potassium fluoride/alumina for 1 mol of the compound of the general formula (XII) in dimethylformamide or acetonitrile at a temperature of from room temperature to about 90°C. According to the improved process, 0.1 to 0.5 equivalent of potassium iodide is further added to the mixture.

In addition, the removal of the amine-protecting group Y gives an amine compound represented by the general formula (III) wherein Y represents a hydrogen atom. A benzyl group as the protecting group can be removed by a hydrogenolysis with palladium/carbon as a catalyst in a solvent such as methanol or by a treatment with hydrogen bromide/acetic acid. When the protecting group Y is an acetyl or trifluoroacetyl group, a treatment with an alkali in a solvent such as methanol gives a compound of the general formula (III) wherein Y represents a hydrogen atom.

In addition, a compound of the general formula (XI) wherein X' is a hydroxyl group can be prepared by a reaction with a compound of the general formula (XII) according to Mitsunobu reaction. That is, there is exemplified a reaction in the presence of 1 to 10 equivalents of triphenylphosphine and 1 to 10 equivalents of diethyl azodicarboxylate in a solvent such as tetrahydrofuran at a temperature of from about 0°C to room temperature.

A compound of the general formula (XI) wherein X' is a hydroxyl group can be prepared by protecting amine of a commercially available amino alcohol with an amine-protecting group Y. The hydroxyl group is then brominated or iodinated according to a conventional method to prepqare the corresponding brominated form or iodinated form. A compound of the general formula (XI) wherein Y is a benzyl group is preferred since it can be easily obtained by brominating a commercially available benzylaminoethanol. Further, if an aminobrominated form is easily available, it can be protected with an amine-protecting group Y to give a compound of the general formula (XI). An exemplified process comprises reacting a commercially available 2-bromoethylamine hydrobromate with benzyloxycarbonyl chloride in the presence of triethylamine in methylene chloride with ice cooling.

In addition, a compound of the general formula (III) can be also obtained by the following process. That is, a compound of the general formula (III) can be obtained by reacting a compound of the general formula (XII) with a compound of the general formula (XIII): 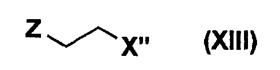 wherein Z represents a leaving group wherein the leaving group means a removable group such as chlorine, bromine or iodine atom, or a sulfonic acid ester such as mesyl or tosyl group, and X" represents a halogen atom, to give a compound of the general formula (XIV): 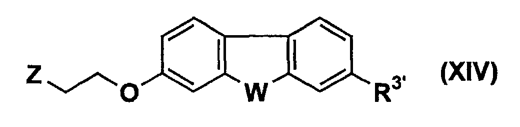 wherein W, Z and R^{3'} are each as defined above; and then replacing Z with a compound of the general formula (XV):

**YNH**_{**2**} **(XV)**

wherein Y represents a hydrogen atom or an amine-protecting group, to give a compound of the general formula (III).

A compound of the general formula (XII) wherein W is a secondary nitrogen atom and R^{3'} is other than hydroxyl, chloro and methyl is a novel compound and can be prepared by the following process. That is, a compound of the general formula (XII) wherein R^{3'} is a hydroxyl group can be prepared according to the process described in S. P. Popri, et al., Indian J. Chem. Sect. B, 14B, p. 371 (1976). This compound can be reacted with alkyl halide in the presence of a base such as potassium carbonate to prepare a compound of the general formula (XII) wherein R^{3'} is OR'. Further, a protecting group can be introduced according to the method for introducing a protecting group set forth above. Further, a compound of the general formula (XII) wherein R^{3'} is a bromine atom or a cyano group can be prepared by deprotecting a compound described in R. R. Tidwell, et al., Eur. J. Med. Chem., 32, p. 781 (1997) under a conventional condition for deprotecting methyl ether. Further, a compound of the general formula (XII) wherein R^{3'} is a chlorine atom can be prepared by deprotecting a compound described in S. P. Popri, et al., J. Med. Chem., 16, p. 425 (1976) likewise in a manner as set forth above. Further, a compound of the general formula (XII) wherein R^{3'} is a lower alkyl group can be prepared by deprotecting as set forth above, a compound prepared according to the process described in R. S. Kapil, et al., Indian J. Chem. Sect. B, 23B, p. 296 (1984). Alternatively, a compound of the general formula (XII) can be obtained by coupling a compound of the general formula (XXVIII): 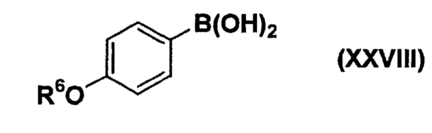 wherein R⁶ represents a hydroxyl-protecting group, with a compound of the general formula (XXIX): 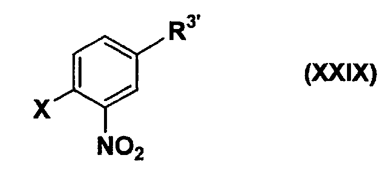 wherein X represents a leaving group, and R^{3'} is as defined above according to Suzuki reaction to give a compound of the general formula (XXX): 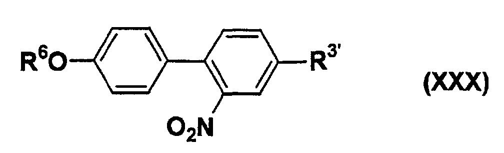 wherein R⁶ and R^{3'} are each as defined above; reductively cyclizing the thus obtained compound of the general formula (XXX) to give a compound of the general formula (XXXI): 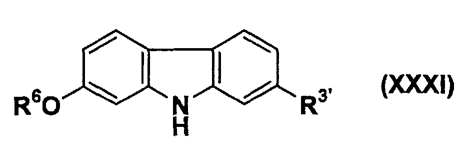 wherein R⁶ and R^{3'} are each as defined above; and then deprotecting the group R⁶.

A compound of the general formula (XXVIII) and a compound of the general formula (XXIX) are commercially available or can be obtained by adding a protecting group to a commercially available compound. Suzuki reaction may be carried out according to the process described in Miyaura Norio, Suzuki Akira, Yuki Gosei Kagaku Kyoukaishi, 46, p. 848 (1988) or the process described in C. W. Holzapfel, et al., Heterocycles, 48, No.8, pp. 1513-1518 (1998).

A compound of the general formula (XXXI) can be prepared according to the process described in J. I. G. Cadogan, et al., J. Chem. Soc., 4831 (1965). That is, a carbazole derivative represented by the general formula (XXXI) can be obtained by heating a compound of the general formula (XXX) in the presence of trialkyl phosphite or triphenyl phosphite to reductively cyclize the compound. The phosphite to be used is preferably triethyl phosphite. It may be used in an amount of 2 to 10 equivalents, preferably 2 to 4 equivalents. The reaction temperature may be in the range of from about 80°C to about 180°C, preferably from about 130°C to about 170°C. The reaction time may be 1 to 24 hours, preferably 3 to 10 hours. Thereafter, R⁶ may be selectively deprotected according to a conventional method to give a compound of the general formula (XII).

In addition, a compound of the general formula (XII) wherein W is an oxygen atom can be obtained by removing the methyl groups of 3,7-dimethoxydibenzofuran described in P. O. Stransky, et al., J. Chem. Soc. Perkin Trans. I, p. 1605 (1982) according to a conventional method and then realkylating or protecting one of the deprotected hydroxyl groups. Further, a compound of the general formula (XII) wherein W is a sulfur atom can be obtained by reducing 3,7-dihydroxydibenzothiophene 5,5-dioxide described in M. M. Joullie, et al., J. Med. Chem., 21, p. 1084 (1978) with lithium aluminum hydride to give 3,7-dihydroxydibenzothiophene, followed by alkylating or protecting treatment as set forth above.

A further alternative process of Preparation Process A may be a process comprising reacting a compound of the general formula (IX): 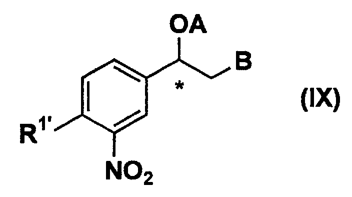 wherein A represents a hydroxyl-protecting group, and R^{1'}, B and * are each as defined above, with a compound of the general formula (III): 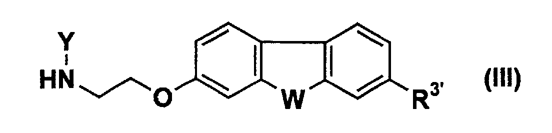 wherein Y represents a hydrogen atom or an amine-protecting group, and W and R^{3'} are each as defined above, to give a compound of the general formula (IV) wherein A represents a hydroxyl-protecting group, and R^{1'}, R^{3'}, W, Y and * are each as defined above; and treating the thus obtained compound according to the method set forth above to give a compound of the general formula (I).

The protecting group A may be introduced and removed according to the method set forth above.

Alternatively, Preparation Process A may be a process comprising reacting a compound of the general formula (VIII): 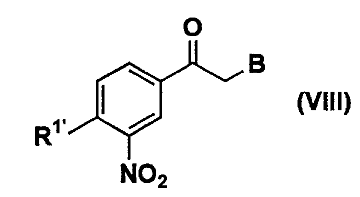 wherein R^{1'} and B are each as defined above, with a compound of the general formula (III) wherein W, Y and R^{3'} are each as defined above, to give a compound of the general formula (XVI): 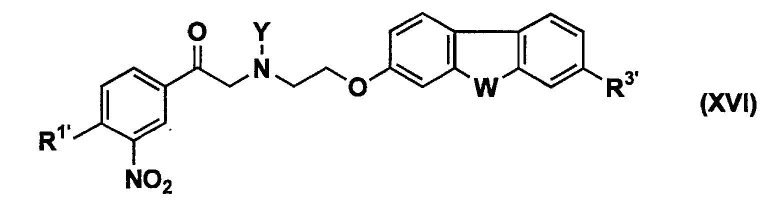 wherein R^{1'}, W, Y and R^{3'} are each as defined above; reducing the carbonyl group according to the method set forth above, to give a compound of the general formula (IV) wherein A represents a hydrogen atom; and treating the thus obtained compound according to the method set forth above, to give a compound of the general formula (I).

The reaction of a compound of the general formula (VIII) with a compound of the general formula (III) is preferably carried out according to a process which improves the process indicated in A. A. Larsen, et al., J. Med. Chem., 10, p. 462 (1967). That is, the process preferably comprises reacting the said compounds in the absence or presence of an amine as an acid-trapping agent in a polar solvent such as acetonitrile, dimethylformamide, dimethylacetamide or dimethylsulfoxide at a temperature of from ice cooling to about 60°C; successively reducing the carbonyl group with a reducing agent such as sodium borohydride or sodium cyanoborohydride at a temperature of from ice cooling to room temperature; and then removing the protecting groups. An optically active substance can be obtained by optical resolution according to the method set forth below, or by asymmetric reduction with a hydrogen donating compound in the presence of the above-mentioned catalyst or a known asymmetric reduction catalyst disclosed in some literatures such as K. Achiwa, et al., Chem. Pharm. Bull., 43, p. 748 (1995) and R. Noyori, et al., J. Am. Chem. Soc., 118, p. 2521 (1996).

An alternative process of Preparation Process A may be a process comprising reacting a compound of the general formula (XVII): 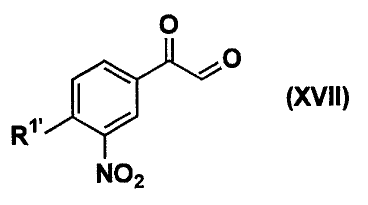 wherein R^{1'} is as defined above, with a compound of the general formula (III) wherein Y represents a hydrogen atom, and W and R^{3'} are each as defined above, followed by reducing the resultant product to give a compound of the general formula (IV) wherein A and Y represent a hydrogen atom, and R^{1'}, R^{3'} and W are each as defined above; and if necessary, and then protecting A and Y by a conventional method, reducing the nitro group likewise by a method set forth above to give a compound of the general formula (V). The compound of the general formula (I) is obtained likewise by a method described above.

This reaction is usually carried out in a medium in the presence of a suitable reducing agent which can reduce Schiff's base obtained from a condensation reaction and can simultaneously reduce carbonyl group to hydroxyl group. Examples of the reducing agent include sodium borohydride, sodium cyanoborohydride, lithium cyanoborohydride and the like. The amount of phenylglyoxal to be used is 1 to 3 mol, preferably 1 to 1.5 mol for 1 mol of the amine. The reaction temperature can be suitably selected and may be generally a temperature of from room temperature to the reflux temperature of the solvent used. The reaction time can be suitably selected depending on the reaction conditions and the reaction may be normally completed when the yield is maximum. An exemplified process is carried out in an alcoholic medium such as methanol or ethanol in the presence of sodium borohydride preferably at a lower temperature. An optically active substance is obtained by optical resolution according to the method set forth below.

A compound of the general formula (XVII) can be easily obtained by oxidizing acetophenone compounds having the substituent R^{1'} with an oxidizing agent such as selenium dioxide in water or an organic solvent which may be a cyclic ether such as dioxane or tetrahydrofuran. Alternatively, the compound can be prepared according to the process indicated in J. Am. Chem. Soc., 79, p. 6562 (1957).

Further, an alternative process of Preparation Process A may be a process comprising reacting an amine compound of the general formula (XVIII): 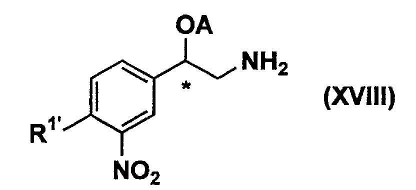 wherein A represents a hydroxyl-protecting group, and R^{1'} and * are each as defined above, with a compound of the general formula (XIV): 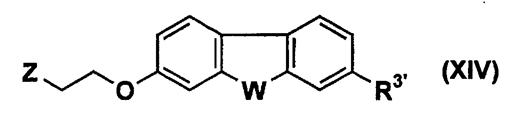 wherein W, R^{3'} and Z are each as defined above, to give a compound of the general formula (IV) wherein Y represents a hydrogen atom, A represents a hydroxyl-protecting group, and R^{1'}, R^{3'} and W are as defined above; and protecting the thus generated amine group, and then preparing a compound of the general formula (I).

The coupling reaction with the amine is carried out in an organic solvent, and if necessary in the presence of a proton acceptor such as a tertiary amine (for example, triethylamine) to give a compound of the general formula (IV) wherein Y represents a hydrogen atom. The leaving group means a group which can be removed in the reaction set forth above, such as chlorine, bromine or iodine atom, or a sulfonic acid ester such as mesyl or tosyl group. The amount of the amine of the general formula (XVIII) to be used as an example of the reaction conditions may be 1 to 10 mol for 1 mol of the compound of the general formula (XIV).

This reaction proceeds slowly and therefore is preferably carried out in an autoclave. Examples of the solvent to be used include alcohols such as methanol, ethanol and butanol, halogenated hydrocarbon such as methylene chloride and chloroform, tetrahydrofuran, dioxan and the like. The reaction temperature is generally in the range of from about 10°C to about 150°C, preferably from about 70°C to about 130°C. The reaction time is generally 5 to 100 hours.

A compound of the general formula (XVIII) may be obtained by hydrogenating a mandelonitrile compound substituted with R^{1'}, for example, in the presence of a catalyst such as Raney nickel. The substituted mandelonitrile may be obtained as a racemic compound by reacting a substituted benzaldehyde with hydrogen cyanide, or with sodium cyanide and sodium hydrogensulfite. The thus obtained racemic compound can be easily resolved into the corresponding optically active isomers by the formation of a salt of diastereomer with a suitably selected optically active acid according to a conventional method and technique. In addition, an optically active compound of the general formula (XVIII) may be obtained by hydrolyzing an optically active substituted mandelonitrile to give an optically active carboxylic acid, and reacting the thus obtained carboxylic acid with ammonia in the presence of a commonly used condensing agent, followed by reducing reaction.

### [Preparation Process B]

Furthermore,an alternative method comprises reacting a compound of the general formula (II) wherein R^{1'} and * are each as defined above, with a compound of the general formula (XI) wherein Y represents an amine-protecting group, and X' represents a hydroxyl group, to give a dialcohol compound of the general formula (XIX): 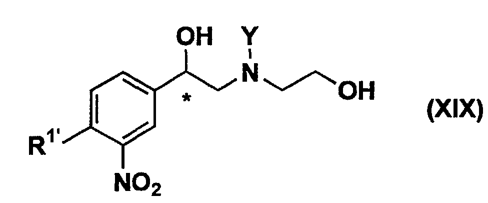 wherein R^{1'}, Y and * are each as defined above; brominating the appropriate primary alcohol moiety; and successively reacting the thus obtained compound with a compound of the general formula (XII) wherein W and R^{3'} are as defined above, to give a compound of the general formula (IV), and then carrying out the reaction in a manner similar to Preparation Process A set forth above to give a compound of the general formula (I).

The reaction of a compound of the general formula (II) with a compound of the general formula (XI) wherein X' represents a hydroxyl group, may be carried out according to the procedure set forth in Preparation Process A.

The primary hydroxyl group of a compound of the general formula (XIX) can be converted into a bromine atom by a bromination reaction with a known brominating agent such as hydrogen bromide/acetic acid, phosphorus tribromide, phosphorus pentabromide, thionyl bromide, bromine/triphenylphosphine, carbon tetrabromide/triphenylphosphine, or N-bromosuccinimide/triphenylphosphine. For example, about 1 to 10 mol of phosphorus tribromide may be reacted for 1 mol of the compound of the general formula (XIX). Generally, this reaction is preferably carried out in an inert medium. The inert medium may be 1,2-dichloroethane, carbon tetrachloride or the like, with dichloromethane being preferred. The amount of the inert medium to be used may be generally about 1 to 10 mL for 1 g of a compound of the general formula (XIX). Generally, this reaction may be preferably carried out at a temperature of from about -30°C to about 100°C, particularly from about 0°C to about 50°C, for example, preferably for 1 to 5 hours.

Generally, the subsequent condensation reaction of the thus brominated compound of the general formula (XIX) with a compound of the general formula (XII) is preferably carried out by reacting 1 to 5 mol of the compound of the general formula (XII) for 1 mol of the brominated compound of the general formula (XIX) under a basic condition. It is preferred that basic condition is achieved by acting a metal alkoxide obtained from an alkali such as potassium carbonate, potassium hydroxide, sodium hydroxide, sodium hydride, potassium hydride, potassium tert-butoxide and the like. The amount of the metal alkoxide to be used may be generally about 1 to 3 mol for 1 mol of the brominated compound of the general formula (XIX). Generally, this reaction is preferably carried out in an inert medium. The inert medium may be acetone, 2-butanone, tetrahydrofuran, N,N-dimethylacetamide, dimethylsulfoxide, sulfolane and the like, with N,N-dimethylformamide being preferred. The amount of the inert medium to be used may be about 1 to 10 mL for 1 g of the brominated form. Generally, this reaction may be preferably carried out at a temperature of from room temperature to about 100°C, for example, preferably for 3 to 10 hours.

A compound of the general formula (XIX) is a novel substance and is useful as an important intermediate for obtaining a compound of the general formula (I).

An alternative process of Preparation Process B may be a process comprising reducing the nitro group of a compound of the general formula (XIX) to give a compound of the general formula (XX): 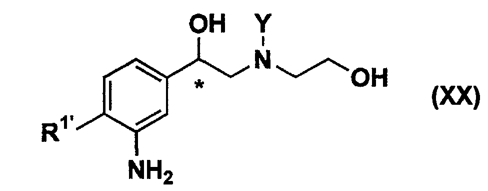 wherein R^{1'}, Y and * are each as defined above; reacting the thus obtained compound with a compound of general formula (VI):

**XSO**_{**2**}**R**^{**2**} **(VI)**

wherein R² represents a lower alkyl group or a benzyl group, and X represents a leaving group, to give a compound of the general formula (XXI): 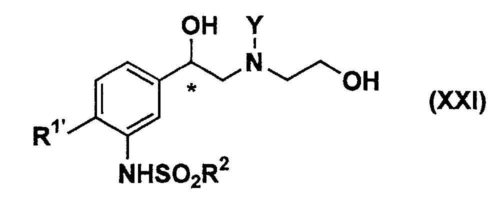 wherein R^{1'}, R², Y and * are each as defined above; successively reacting the thus obtained compound with a compound of the general formula (XII) wherein W and R^{3'} are as defined above, to give a compound of the general formula (VII) wherein A represents a hydrogen atom; and then simultaneously or sequentially removing the protecting groups to give a compound of the general formula (I).

The reduction of a compound of the general formula (XIX) wherein Y is a hydrogen atom may be carried out according to the above process comprising first protecting the compound and then reducing the nitro group. The thus obtained compound of the general formula (XX) is a novel substance and is an important intermediate for obtaining a compound of the general formula (I).

The sulfonation of the amine (aniline) of a compound of the general formula (XX) may be carried out according to the process set forth above. The thus obtained compound of the general formula (XXI) is also a novel substance and is an important intermediate for obtaining a compound of the general formula (I). The compound of the general formula (XXI) is then subjected to a condensation reaction with a compound of the general formula (XII) in a manner described above and then simultaneously or sequentially removing the protecting groups of R^{1'}, Y and R^{3'} to give a compound of the general formula (I).

### [Preparation Process C]

A further alternative process comprises chlorinating a compound of the general formula (XXII): 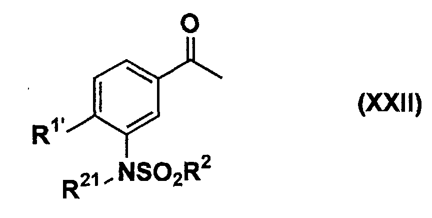 wherein R^{1'} and R² are each as defined above, and R²¹ represents an amine-protecting group, to give a compound of the general formula (XXIII): 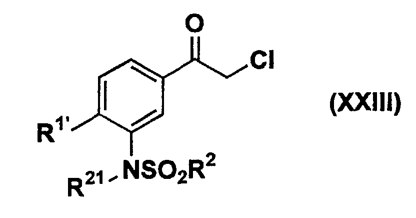 wherein R^{1'}, R² and R²¹ are each as defined above; reducing the thus obtained compound to give a compound of the general formula (XXIV): 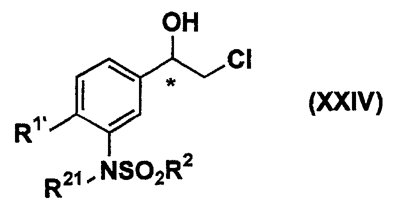 wherein R^{1'}, R², R²¹ and * are each as defined above; subjecting the thus obtained compound to an alkali treatment to give a compound of the general formula (XXV): 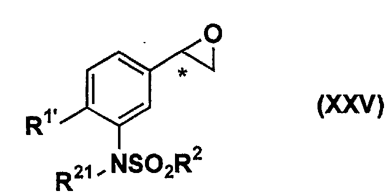 wherein R^{1'}, R², R²¹ and * are each as defined above; reacting the thus obtained compound with a compound of the general formula (III) wherein W, Y and R^{3'} are each as defined above to give a compound of the general formula (XXVI): 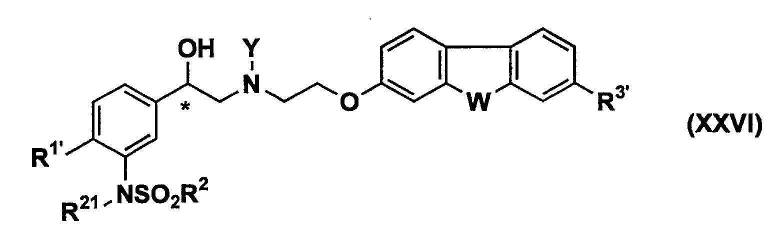 wherein R^{1'}, R², R^{3'}, R²¹, W, Y and * are each as defined above; and then simultaneously or sequentially removing the protecting groups existing in R^{1'}, R^{3'}, R²¹ and Y, to give a compound of the general formula (I).

A compound of the general formula (XXII) may be prepared by introducing according to the method set forth above an amine-protecting group R²¹ to 4'-R^{1'}-3'-methylsulfonylaminoacetophenone which can be prepared by a method described in literatures (for example, A. A. Larsen, et al., J. Med. Chem., 10, p. 462 (1967); C. Kaiser, et al., J. Med. Chem., 7, p. 49 (1974); or JP-A-9-249623 (WO 97/25311).

A compound of the general formula (XXIII) is a novel substance and may be obtained by chlorinating a compound of the general formula (XXII) set forth above. The chlorinating process may be carried out using a conventionally used chlorinating agent. A compound of the general formula (XXIII) may be also prepared by a method described in literatures (for example, D. Masilamani, et al., J. Org. Chem., 46, p. 4486 (1981). For example, the chlorinating agent may be sulfuryl chloride. That is, a compound of the general formula (XXII) may be chlorinated by reaction with sulfuryl chloride in the presence of methanol in an organic solvent such as methylene chloride or toluene.

A compound of the general formula (XXIV), which is novel and relatively good in crystallinity, is characteristic as an important intermediate.

A compound of the general formula (XXIV) may be obtained by reducing a compound of the general formula (XXIII) set forth above with a known reducing agent. The reducing agent may be sodium borohydride, borane, diisobutylaluminum hydride or the like. A compound of the general formula (XXIII) may be preferably reduced with a metal hydride such as sodium borohydride, or with hydrogen in the presence of the platinum group metal catalyst such as a palladium catalyst. The amount of sodium borohydride to be added may be generally about 1 to 3 mol for 1 mol of the compound of the general formula (XXIII). Generally, this reaction is preferably carried out in a lower alcohol. The lower alcohol may be methanol, i-propanol or the like, with ethanol being preferred. The amount of the lower alcohol to be used may be generally about 1 to 5 mL for 1 g of the compound of the general formula (XXIII). When the solubility is insufficient, it may be preferred that tetrahydrofuran as a cosolvent be generally added in an amount of about 1 to 5 mL for 1 g of the compound of the general formula (XXIII). Generally, this reaction is preferably carried out at a temperature of from about -20°C to about 50°C, particularly from about 0°C to room temperature, for example, for 1 to 5 hours.

In addition, if an optical isomer of either R-form or S-form with respect to * of the general formula (XXIV) is to be obtained, it can be obtained by asymmetric reduction with a hydrogen donating compound in the presence of an asymmetric reduction catalyst set forth above.

A compound of the general formula (XXV) is a novel substance with a good crystallinity. The said compound, which can be purified by recrystallization and can be used to improve the optical purity, is a useful intermediate. A compound of the general formula (XXV) can be obtained from a compound of the general formula (XXIV) by a conventional process. An exemplified process may comprise reacting a compound of the general formula (XXIV) in the presence of 1 to 5 mol of alkali for 1 mol of the compound in a solvent such as an alcoholic solvent (such as methanol or ethanol) or acetone at a temperature of from room temperature to the reflux temperature of the solvent to be used. Examples of the alkali include sodium carbonate, potassium carbonate, sodium hydroxide and potassium hydroxide.

A compound of the general formula (XXVI) can be prepared from a compound of the general formula (XXV) and a compound of the general formula (III) according to the process set forth in Preparation Process A. A compound of the general formula (I) can be obtained by subjecting a compound of the general formula (XXVI) to the deprotecting treatment set forth above.

### [Preparation Process D]

A further alternative process comprises reacting a compound of the general formula (XXV) with a compound of the general formula (XI) wherein Y represents an amine-protecting group, and X' represents a hydroxyl group, to give a compound of the general formula (XXVII): 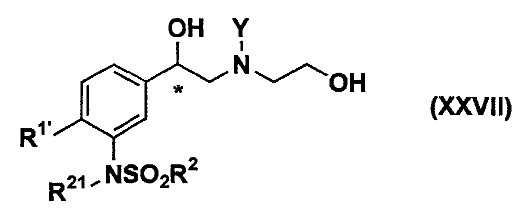 wherein R^{1'}, R², R²¹, Y and * are each as defined above; brominating the thus obtained compound as set forth above, reacting the brominated compound with a compound of the general formula (XII) to give a compound of the general formula (XXVI); and deprotecting according to Preparation Process C to give a compound of the general formula (I).

A variety of compounds described herein may be purified, if necessary, and such a purification can be usually carried out by a known chromatography (column, flash column, thin layer, or high-performance liquid chromatography) with referring to, for example, Rf values indicated in the present text of specification.

As mentioned above, a compound of the general formula (I) can exist in the form of either of two optical isomers. The process of the present invention can provide both pure optical isomers and a racemic mixture. The reactions set forth above do not alter the stereochemistry involved in such reactions at all.

Therefore, a racemic modification can be obtained by a process starting from a compound of the general formula (VIII), (XVII) or (XXII) which contains no asymmetric carbon, or from a compound of the general formula (II), (IX), (XVIII), (XXVI) or (XXV) as a racemic compound. Likewise, starting from an optically pure isomer of a compound of the general formula (II), (IX), (XVIII), (XXVI) or (XXV), for example, R-isomer of the general formula (II), only R-isomer is obtained. Further, a pure isomer can be obtained using an optically active isomer of a compound of the general formula (II), (IX), (XVIII), (XXVI) or (XXV).

When a mixture of two enantiomers (racemic modification) is obtained, it can be optically resolved by a suitable method such as a method comprising fractionally crystallizing the enantiomers as acid addition salts with an optically active acid such as camphorsulfonic acid, mandelic acid or substituted mandelic acid. Such a fractional crystallization may be carried out using a suitable solvent, preferably a lower alkanol, such as ethanol, isopropanol or a mixture thereof.

Each pair of enantiomers can be resolved into pure isomers by formation of diastereomeric salt, chromatography using an optically active column, or other means. When one of starting materials is optically active, the thus obtained mixture of diastereomers can be resolved into pure isomers by the above-mentioned means. Isolation and purification of an optically active isomer makes possible enhanced efficiency and dissolution of side effects due to the use of higher active isomer to give a preferred drug.

Salts of a compound of the general formula (I) may be a known salt, and examples thereof include hydrochloride, hydrobromate, sulfate, hydrogensulfate, dihydrogen phosphate, citrate, maleate, tartrate, fumarate, gluconate, methanesulfonate and the like, and acid addition salts with an optically active acid such as camphorsulfonic acid, mandelic acid or substituted mandelic acid. Among them, pharmaceutically acceptable salts are particularly preferred.

When a compound of the general formula (I) is converted into its salt, an acid addition salt of the compound can be obtained by dissolving the compound in alcohol such as methanol or ethanol to which the equivalent amount to several times amount of the acid component is added. The acid component to be used may be a pharmaceutically acceptable mineral or organic acid, such as hydrochloric acid, hydrobromic acid, sulfuric acid, hydrogensulfate, dihydrogen phosphate, citric acid, maleic acid, tartaric acid, fumaric acid, gluconic acid or methanesulfonic acid.

Tricyclic compounds of the present invention and pharmaceutically acceptable salts thereof, which have no recognizable toxic effect, are useful as a medicine. For example, the compounds, which have β3-agonist activities, can be used as a medicine for treating and preventing β3-associated diseases. The term "β3-associated disease" is a generic term directed to diseases which can be improved by agonistic effects mediated by β3-adrenoreceptor. Examples of β3-associated diseases include diabetes, obesity, hyperlipidemia, digestive diseases (preferably dyskinesis of digestive system or ulcer) and depression. According to the present invention, the preferred examples include diabetes, obesity and hyperlipidemia. That is, the present compounds are useful as a medicine for treating and preventing diabetes, since they are expected to exhibit hypoglycemic activity. The present compounds are also useful as a medicine for treating and preventing hyperlipidemia and as a medicine for treating obesity, since they are expected to exhibit lipolytic activity.

Even tricyclic compounds of the present invention and pharmaceutically acceptable salts thereof obtained by a synthetic means have β3-agonistic effects, and those generated as a result of an in vivo metabolism also have the same β3-agonist activity. Therefore, compounds which generate the present compound as a result of an in vivo metabolism are also useful as therapeutic agents set forth above.

A medicine of the present invention is preferably prepared in the form of a pharmaceutical composition by optionally adding a pharmaceutically acceptable carrier to an effective amount of a tricyclic compound represented by the general formula (I) or a salt thereof. Examples of pharmaceutically acceptable carriers include excipients, binders such as carboxymethylcellulose, disintegrators, lubricants and auxiliaries.

When a compound of the present invention is administered to humans, it can be orally administered in the form of tablet, powder, granule, capsule, sugar-coated tablet, solution, syrup or the like. Further, it can be parenterally administered in the form of injection or the like. The dosage administered will vary dependent on the age and weight of the patient and the extent of disease. The daily dosage for an adult is usually 0.01 to 2000 mg, which is singly administered or is divided into several dosages and then administered. The administration period can vary between several weeks and several months and the everyday medication is usually applied. However, the daily dosage and administration period can be increased or decreased from the above ranges dependent on the conditions of patient.

### Examples

The following examples further illustrate this invention but are not intended to limit it in any way.

The thin layer chromatography (TLC) used was Precoated silica gel 60 F₂₅₄ (mfd. by Merck). After developing with chloroform/methanol (1:0 to 4:1), chloroform/acetone (1:0 to 10:1), or n-hexane/ethyl acetate (1:0 to 1:10), the detecting process was carried out with UV (254 nm) irradiation and coloration with ninhydrin. Rf values of TLC are shown on free amines. The organic layers were dried over anhydrous magnesium sulfate or anhydrous sodium sulfate. The silica gel column chromatography process was carried out on silica gel 60 (230-400 mesh; mfd. by Merck). The determination of nuclear magnetic resonance spectrum (NMR) was carried out using Gemini-300 (FT-NMR; mfd. by Varian). A solvent used was specified in each example. Tetramethylsilane was used as the internal standard and NMR data are indicated herein in δ (ppm).

In this connection, the splitting patterns are indicated using the following abbreviations.

| | | | |
|---|---|---|---|
| s | singlet; | d | doublet; |
| t | triplet; | q | quartet; |
| m | multiplet; | dd | double doublet; |
| br | broad singlet. | | |

Mass spectrum (MS) was determined by the fast atom bombardment mass spectrometry (FAB-MS) with JEOL-JMS-SX102.

### [Intermediate 1]

### Preparation of 2-hydroxy-7-trifluoromethyl-9H-carbazole

### A. Preparation of N-acetyl-2-bromo-5-trifluoromethylaniline

2-Bromo-5-trifluoromethylaniline (5.04 g) was added to pyridine (20 mL), which was then cooled with ice. Acetic anhydride (2 mL) was added dropwise and the resulting mixture was stirred for 17 hours while it was slowly brought back to room temperature. Acetic anhydride (1 mL) was further added and the mixture was stirred at 80°C for 4 hours. The reaction liquid was brought back to room temperature and methylene chloride was added. The resulting mixture was washed with aqueous 1 N hydrochloric acid (twice), saturated sodium bicarbonate water and saturated brine. The organic layer was dried and the solvent was distilled off under reduced pressure. The residue was recrystalized from ethanol to yield the title compound (3.42 g) as a white crystal.
Rf=0.61 (1:1 hexane/ethyl acetate);
¹H-NMR (CDCl₃): 8.71 (1H, s) , 7.64-7.67 (2H, m), 7.21-7.26 (1H, m), 2.27 (3H, s);
Mass (m/e): 283 (MH⁺).

### B. Preparation of N-acetyl-2-(4-methoxyphenyl)-5-trifluoromethylaniline

4-Methoxyphenylboronic acid (6.35 g) was dissolved in toluene (65 mL), to which was added a compound (5.89 g; prepared according to the procedure of the step A of Intermediate 1). Tetrakistriphenylphosphine palladium(0) (1.21 g) and potassium carbonate (17.33 g) were added and the resulting mixture was stirred at 90°C for 3.5 hours. The reaction liquid was cooled to room temperature. Aqueous 2 N hydrochloric acid (200 mL) was added and the reaction liquid was extracted with ethyl acetate. The organic layer was washed with aqueous 1 N hydrochloric acid and saturates brine, and dried. The solvent was distilled off under reduced pressure and the residue was purified by silica gel column chromatography (4:1 hexane/ethyl acetate) to yield the title compound (5.89 g).
Rf=0.54 (1:1 hexane/ethyl acetate);
¹H-NMR(CDCl₃): 8.66 (1H, br), 7.23-7.42 (5H, m), 7.03-7.07 (2H, m), 3.89 (3H, s), 2.05 (3H, s) ;
Mass (m/e): 310 (MH⁺).

### C. Preparation of 2-(4-methoxyphenyl)-5-trifluoromethylaniline hydrochloride

A compound (5.80 g; prepared according to the procedure of the step B of Intermediate 1) was suspended in a mixed solvent of ethanol (50 mL) and concentrated hydrochloric acid (50 mL). The mixture was refluxed for 1 hour. The reaction liquid was cooled to room temperature and ethanol in the mixed solvent was distilled off under reduced pressure. A white suspension of the residue was filtered and the precipitate was dried in vacuo to yield the title compound (4.93 g).
Rf=0.76 (1:1 hexane/ethyl acetate);
¹H-NMR (DMSO-d₆): 8.45 (3H, br), 7.05-7.47 (7H, m), 3.82 (3H, s);
Mass (m/e): 268 (MH⁺).

### D. Preparation of 2-(4-methoxyphenyl)-5-trifluoromethylazobenzene

A compound (4.80 g; prepared according to the procedure of the step C of Intermediate 1) was added to a mixed solvent of water (24 mL) and concentrated hydrochloric acid (8 mL) and the resulting mixture was stirred with ice cooling. A solution of sodium nitrite (1.37 g) in water (5 mL) was added dropwise with stirring over 8 minutes and the mixture was further stirred for 30 minutes. A solution of sodium azide (1.17 g) in water (5 mL) was then added dropwise over 5 minutes and the mixture was further stirred for 12 minutes. Methylene chloride (120 mL) was added. The organic layer was washed with water and then dried. The solvent was distilled off under reduced pressure and the residue was purified by silica gel column chromatography (9:1 hexane/ethyl acetate) to yield the title compound (3.50 g).
Rf=0.84 (2:1 hexane/ethyl acetate);
¹H-NMR (CDCl₃): 7.37-7.45 (5H, m), 6.97-7.00 (2H, m), 3.86 (3H, s);
Mass (m/e): 294 (MH⁺).

### E. Preparation of 2-methoxy-7-trifluoromethyl-9H-carbazole

A compound (3.4 g; prepared according to the procedure of the step D of Intermediate 1) was dissolved in decalin (200 mL), which was stirred at 200°C for 1.5 hours. After the reaction was completed, the reaction liquid was cooled to room temperature and further cooled with ice. The generated precipitate was collected by filtration, washed with hexane and then dried in vacuo to yield the title compound (1.84 g).
Rf=0.77 (1:1 hexane/ethyl acetate);
¹H-NMR (DMSO-d₆): 11.48 (1H, br), 8.20 (1H, d, J=8.4), 8.09 (1H, d, J=8.7), 7.74-7.75 (1H, m), 7.40-7.44 (1H, m), 7.06 (1H, d, J=2.4), 6.85 (1H, dd, J=8.7, 2.4), 3.87 (3H, s);
Mass (m/e): 266 (MH⁺).

### F. Preparation of 2-hydroxy-7-trifluoromethyl-9H-carbazole

A compound (1.79 g; prepared according to the procedure of the step E of Intermediate 1) was mixed with pyridine hydrochloride (5 g) and the resulting mixture was stirred at 230°C for 30 minutes. The reaction liquid was cooled to room temperature and water (100 mL) was then added with stirring to precipitate a crude crystal. The precipitate was filtered and washed with ethanol to yield a crude product (1.64 g) of the title compound. This crude product was washed with chloroform (50 mL) three times and then with chloroform (10 mL). The resulting solid was dissolved in THF (10 mL), to which aqueous 1 N hydrochloric acid (3 mL) was added. The resulting mixture was stirred for few minutes and then poured into water, which was then vigorously stirred. The precipitate was filtered and dried in vacuo at 60°C to yield the title compound (500 mg).
Rf=0.51 (1:1 hexane/ethyl acetate);
¹H-NMR (DMSO-d₆): 11.31 (1H, br), 9.65 (1H, br), 8.13 (1H, d, J=8.1), 7.97 (1H, d, J=8.4), 7.67 (1H, d, J=0.6), 7.39 (1H, dd, J=8.1, 0.6), 6.88 (1H, d, J=1.8), 6.71 (1H, dd, J=8.4, 1.8);
Mass (m/e): 252 (MH⁺).

### [Example 1]

### Preparation of 2-(7-trifluoromethyl-9H-carbazol-2-yloxy)ethylamine hydrobromate

### A. Preparation of N-benzyloxycarbonyl-2-(7-trifluoromethyl-9H-carbazol-2-yloxy)ethylamine

N-benzyloxycarbonyl-2-bromoethylamine (645 mg), potassium carbonate (1.36 g) and a compound (496 mg; prepared according to the procedure of the step F of Intermediate 1) were added to DMF (7 mL). The resulting mixture was stirred at 60°C for 17 hours. The reaction liquid was cooled to room temperature, and diluted with water. The precipitated product was filtered, washed with ether and dried to yield the title compound (307 mg). In addition, the filtrate was extracted with ethyl acetate and the organic layer was washed sequentially with an aqueous 2 N sodium hydroxide solution and water, and dried. The solvent was then distilled off under reduced pressure. The residue was purified by silica gel column chromatography (100:0 to 99:1 chloroform/methanol). The residue was recrystalized from chloroform/ethanol to yield the title compound (92 mg)(Total amount: 399 mg).
Rf=0.62 (10:1 chloroform/methanol);
¹H-NMR(DMSO-d₆): 11.48 (1H, br), 8.21 (1H, d, J=8.1), 8.09 (1H, d, J=8.7), 7.75 (1H, s), 7.51-7.57 (1H, m), 7.42 (1H, d, J=7.2), 7.30-7.37 (5H, m), 7.07 (1H, s), 6.85 (1H, d, J=8.7), 5.05 (2H, s), 4.10 (2H, t, J=5.7), 3.41-3.50 (2H, m);
Mass (m/e): 429 (MH⁺).

### B. Preparation of 2-(7-trifluoromethyl-9H-carbazol-2-yloxy)-ethylamine hydrobromate

A compound (392 mg; prepared according to the procedure of the step A of Example 1) was dissolved in a 30% hydrobromic acid/acetic acid solution (1.8 mL). The resulting reaction liquid was stirred at room temperature for 2 hours. Diethyl ether (10 mL) was added. The reaction liquid was stirred for 20 minutes and then filtered. The resulting solid was washed with diethyl ether (5 mL) twice and dried under reduced pressure at 40°C for 2 hours to yield the title compound (312 mg).
¹H-NMR(DMSO-d₆): 11.56 (1H, br), 8.24 (1H, d, J=8.00), 8.15 (1H, d, J=8.5), 8.05 (3H, br), 7.78 (1H, s), 7.44 (1H, d, J=8.2), 7.13 (1H, d, J=2.2), 7.93 (1H, dd, J=8.8, 2.2), 4.29 (2H, t, J=4.9), 3.30 (2H, t, J=4.9);
Mass (m/e): 295 (MH⁺).

### [Intermediate 2]

### Preparation of 7-benzyloxy-2-hydroxy-9H-carbazole

### A. Preparation of 2,7-dihydroxy-9H-carbazole

2,7-Dimethoxy-9H-carbazole (1.94 g; prepared according to the process described in M. H. Litt, et al., Macromolecules, 18, p. 1388 (1985)) was mixed with pyridine hydrochloride (6.39 g) and the resulting mixture was stirred at 232°C for 20 minutes. The reaction liquid was cooled to room temperature. Water was added to the mixture to precipitate a crude product, which was then washed with water and dried under reduced pressure at 60°C to yield the title compound (1.50 g).
Rf=0.27 (1:1 hexane/ethyl acetate);
¹H-NMR (CDCl₃): 10.61 (1H, br), 9.13 (2H, s), 7.64 (2H, d, J=8.3), 6.71 (2H, d, J=2.0), 6.53 (2H, dd, J=8.3, 2.0);
Mass (m/e): 199 (M⁺).

### B. Preparation of 2-benzyloxy-7-hydroxy-9H-carbazole

Potassium carbonate (832.7 mg) was added to a compound (1.00 g; prepared according to the procedure of the step A of Intermediate 2) dissolved in DMF (50 mL). The reaction liquid was cooled to 0°C and a solution of benzyl bromide (944.8 mg) in DMF (5mL) was added dropwise from a dropping funnel over 5 minutes. The dropping funnel was washed with DMF (2.5 mL; twice) and then stirred at 0°C for 3 hours. After the reaction was quenched with an aqueous 2 N sodium hydroxide solution, the organic layer was washed with hexane three times. Aqueous 6 N hydrochloric acid was added to the aqueous layer to give an aqueous solution (pH 3), which was then extracted with ethyl acetate three times. The organic layer was washed with saturated brine and then dried. After the solvent was distilled off under reduced pressure, the residue was purified by silica gel column chromatography (100:0 to 1:2 hexane/ethyl acetate) to yield the title compound (138.1 mg). In addition, 80% of the starting material was recovered.
Rf=0.71 (1:2 hexane/ethyl acetate);
¹H-NMR (DMSO-d₆): 10.74 (1H, br), 9.17 (1H, br), 7.71 (1H, d, J=8.6), 7.65 (1H, d, J=8.3), 7.24-7.43 (5H, m), 6.88 (1H, s), 6.70 (1H, d, J=8.6), 6.69 (1H, s), 6.51 (1H, d, J=8.3), 5.09 (2H,s);
Mass (m/e): 289 (M⁺).

### [Intermediate 3]

### Preparation of (R)-1-[3-(N-benzyl-N-methylsulfonylamino)phenyl] oxirane

### A. Preparation of 3'-(N-benzyl-N-methylsulfonylamino)acetophenone

Potassium carbonate (884 g), benzyl bromide (254 mL) and sodium iodide (176 g) were added to a solution of 3'-(methylsulfonylamino)acetophenone (227 g; prepared by the process reported by A. A. Larsen, et al., J. Med. Chem., 10, p. 462 (1967) or C. Kaiser, et al., J. Med. Chem., 7, p. 49 (1974), or by the process described in JP-A-9-249623 (WO97/25311)) in dimethylformamide (2.14 L). The resulting mixture was stirred at room temperature for 12.2 hours. The reaction liquid was poured into water (10 L) and stirred for 1 hour. The precipitated brown solid was collected by filtration and dissolved in ethyl acetate (2 L). The resulting solution was concentrated under reduced pressure and the residue was dissolved in hot toluene. The insoluble matter was then filtered off to give the filtrate 1. The aqueous layer was extracted with ethyl acetate (8 L), dried, concentrated and combined with the filtrate 1. The mixture was concentrated and crystallized from toluene (500 mL) and heptane (150 mL). The precipitated solid was collected by filtration, washed with heptane (300 mL) three times and then dried under reduced pressure at room temperature to give the title compound (281 g) as a light yellow solid.
Rf=0.32 (1:1 hexane/ethyl acetate);
¹H-NMR (CDCl₃): 7.82-7.86 (2H, m), 7.38-7.48 (2H, m), 7.24-7.27 (5H, m), 4.89 (2H, s), 2.98 (3H, s), 2.55 (3H,s);
Mass (m/e): 304 (MH⁺).

### B. Preparation of 2-chloro-1-[3-(N-benzyl-N-methylsulfonylamino)phenyl]ethanone

A solution of sulfuryl chloride (0.46 mL) in methylene chloride (3.3 mL) was added dropwise to a solution of a compound (1 g; prepared according to the procedure of the step A of Intermediate 3) in methylene chloride (1.65 mL) and methanol (0.53 mL) at room temperature over 1 hour. After the reaction was completed, water (10 mL) was added and the layers were separated. The organic layer was washed with an aqueous 0.1 N sodium hydroxide solution (10 mL) three times, dried and concentrated under reduced pressure. The residue was purified by silica gel column chromatography (2:1 hexane/ethyl acetate) to yield the title compound (987 mg) as a colorless solid.
Rf=0.48 (1:1 hexane/ethyl acetate);
¹H-NMR (CDCl₃): 7.82-7.85 (2H, m), 7.43-7.54 (2H, m), 7.21-7.31 (5H, m), 4.89 (2H, s), 4.62 (2H, s), 2.99 (3H, s);
Mass (m/e): 338 (MH⁺).

### C. Preparation of (R)-2-chloro-1-[3-(N-benzyl-N-methylsulfonylamino)phenyl]ethanol

[(S,S)-N-(p-toluenesulfonyl)-1,2-diphenylethylenediamine] (p-cymene) ruthenium complex (63.6 mg; prepared according to the method reported by R. Noyori, et al., J. Am. Chem. Soc., 118, p. 2521 (1996)) was added to a solution of a compound (3.38 g; prepared according to the procedure of the step B of Intermediate 3) in a formic acid/triethylamine solution (5 mL; 5:2 formic acid/triethylamine complex; mfd. by FLUKA) and tetrahydrofuran (5 mL). The resulting mixture was stirred at room temperature for 4 hours.

After the reaction was completed, ethyl acetate (30 mL) and water (30 mL) were added to the reaction liquid, which was then stirred vigorously. The layers were separated. The organic layer was washed with saturated brine (30 mL), and then dried. After the solvent was distilled off under reduced pressure, the residue was purified by silica gel column chromatography (3:1 hexane/ethyl acetate) to yield the title compound (3.51 g).
Rf=0.40 (1:1 hexane/ethyl acetate);
¹H-NMR (CDCl₃): 7.20-7.36 (9H, m), 4.82-4.86 (3H, m), 3.64 (1H, dd, J=11.3, 3.5), 3.52 (1H, dd, J=11.3, 8.2), 2.95 (3H, s), 2.77 (1H, d, J=3.3);
Mass (m/e): 340 (MH⁺);
HPLC: Retention Time (R-form: 62.9 min (S-form: 67.7 min))
Column: CHIRALCEL™ OD-RH (mfd. by Daicel; 4.6 mm ID × 150 mm);
Solvent: 75:25 0.1 M KPF₆/acetonitrile;
Flow rate: 0.5 mL/min;
Detecting wave length: 254 nm;
Temperature: 40°C.

### D. Preparation of (R)-1-[3-(N-benzyl-N-methylsulfonylamino)phenyl]oxirane

Potassium carbonate (1.23 g) was added to a solution of a compound (1.52 g; prepared according to the procedure of the step C of Intermediate 3) in acetone (15.2 mL). The resulting mixture was stirred at the reflux temperature for 5 hours and cooled to room temperature. Ethyl acetate (50 mL) and water (50 mL) were then added and the layers were separated. The aqueous layer was extracted with ethyl acetate. The extract combined with the organic layer separated above was dried and the solvent was distilled off under reduced pressure. The residue was purified by silica gel column chromatography (3:1 hexane/ethyl acetate) to yield the title compound (700 mg), which was recrystallized from methanol.
Rf=0.47 (1:1 hexane/ethyl acetate);
¹H-NMR (CDCl₃): 7.16-7.32 (9H, m), 4.79-4.90 (2H, m), 3.80 (1H, dd, J=4.1, 2.5), 3.12 (1H, dd, J=5.5, 4.1), 2.94 (3H, s), 2.67 (1H, dd, J=5.5, 2.5);
Mass (m/e): 304 (MH⁺);
HPLC: Retention Time (R-form: 92.9 min (S-form: 100.1 min))
Column: CHIRALCEL™ OB-H (mfd. by Daicel; 4.6 mm ID × 250 mm);
Solvent: 9:1 hexane/ethanol;
Flow rate: 0.5 mL/min;
Detecting wave length: 254 nm;
Temperature: 40°C.

### [Example 2]

### Preparation of (R)-N-[3-[2-[2-(7-hydroxy-9H-carbazol-2-yloxy)ethylamino]-1-hydroxyethyl]phenyl]methanesulfonamide hydrochloride

### A. Preparation of (R)-N-benzyl-N-[3-[2-(N'-benzyl-2-hydroxyethylamino)-1-hydroxyethyl]phenyl]methanesulfonamide

A compound (1.99 g; prepared according to the procedure of the step D of Intermediate 3) was dissolved in N-benzylethanolamine (3.01 g), which was then heated at 100°C for 1 hour with stirring. The reaction liquid was cooled to room temperature and purified by silica gel column chromatography (100:0 to 50:1 chloroform/methanol) to yield the title compound (3.41 g).
Rf=0.35 (19:1 chloroform/methanol);
¹H-NMR (CDCl₃): 7.10-7.37 (14H, m), 4.82 (2H, s), 4.61 (1H, dd, J=9.3, 3.9), 3.83 (1H, d, J=13.5), 3.54-3.72 (3H, m), 2.91 (3H, s), 2.76-2.86 (1H, m), 2.61-2.71 (2H, m), 2.55 (1H, dd, J=13.5, 9.3);
Mass (m/e): 454 (M⁺).

### B. Preparation of (R)-N-benzyl-N-[3-[2-(N'-benzyl-2-bromoethylamino)-1-hydroxyethyl]phenyl]methanesulfonamide

A compound (998.2 mg; prepared according to the procedure of the step A of Example 2) was dissolved in methylene chloride (22 mL), which was then cooled to -15°C. Triphenylphosphine (576.1 mg) dissolved in methylene chloride (5 mL) was added dropwise over 2 minutes. The resulting mixture was stirred for 10 minutes and then N-bromosuccinimide (391.1 mg) was added. The mixture was stirred for 30 minutes and the reaction was quenched with methanol. After the solvent was distilled off under reduced pressure, the residue was purified by silica gel column chromatography (100:0 to 3:2 hexane/ethyl acetate) to yield the title compound (749.0 mg). Rf=0.66 (2:1 hexane/ethyl acetate);
¹H-NMR (CDCl₃): 7.13-7.39 (14H, m), 4.82 (2H, s), 4.59 (1H, dd, J=10.4, 3.3), 3.57-3.86 (2H, m), 3.38-3.42 (2H, m), 2.92 (3H, s), 2.85-3.09 (2H, m), 2.66-2.71 (1H, m), 2.40-2.48 (1H, m);
Mass (m/e): 518 (MH⁺).

### C. Preparation of (R)-N-benzyl-N-[3-[2-[N'-benzyl-2-(7-benzyloxy-9H-carbazol-2-yloxy)ethylamino]-1-hydroxyethyl]phenyl]methanesulfonamide

A compound (100.2 mg; prepared according to the procedure of the step B of Intermediate 2) was dissolved in THF (1.7 mL) and an aqueous 2 N sodium hydroxide solution was added. To this reaction liquid a solution of a compound (457.2 mg; prepared according to the procedure of the step B of Example 2) in THF (1.7 mL) was added. The resulting mixture was stirred at room temperature for 30 minutes. The reaction liquid was extracted with ethyl acetate three times and the organic layer was then washed with saturated brine and dried. After the solvent was distilled off under reduced pressure, the residue was purified by silica gel column chromatography (1:1 hexane/ethyl acetate) to yield the title compound (205.8 mg).
Rf=0.34 (1:1 hexane/ethyl acetate);
¹H-NMR (CDCl₃): 8.07 (1H, s), 7.84 (1H, d, J=8.2), 7.82 (1H, d, J=8.6), 7.09-7.64 (19H, m), 6.96 (1H, d, J=2.0), 6.90 (1H, dd, J=8.2, 2.0), 6.89 (1H, d, J=2.3), 6.80 (1H, dd, J=8.6, 2.3), 5.15 (2H, s), 4.79 (1H, s), 4.67 (1H, dd, J=9.9, 3.3), 4.1 (2H, ddd, J=10.6, 9.9, 3.3), 3.96 (1H, d, J=13.5), 3.69 (1H, d, J=13.5), 3.08 (1H, m), 3.00 (1H, m), 2.89 (3H, s), 2.82 (1H, dd, J=12.9, 3.3), 2.60 (1H, dd, J=12.9, 10.2);
Mass (m/e): 726 (MH⁺).

### D. Preparation of (R)-N-[3-[2-[2-(7-hydroxy-9H-carbazol-2-yloxy)ethylamino]-1-hydroxyethyl]phenyl]methanesulfonamide hydrochloride

A compound (100.2 mg; prepared according to the procedure of the step C of Example 2) was dissolved in a mixed solvent of methanol (2.3 mL), THF (2.3 mL) and acetic acid (0.1 mL) under an argon atmosphere, and 20% palladium hydroxide/carbon (49.2 mg) was then added. After replacing the argon stream with hydrogen gas, the mixture was stirred at room temperature for 16 hours. The reaction mixture was filtered to separate the 20% palladium hydroxide/carbon and the residue was then washed with hot methanol. The washings were combined with the filtrate and the solvent was distilled off under reduced pressure. The residue was dissolved in a mixed solvent of methanol (4.6 mL) and acetic acid (0.1 mL) under an argon atmosphere again, and 20% palladium hydroxide/carbon (100.2 mg) was then added. After the atmosphere was replaced with hydrogen gas, the resulting mixture was stirred at room temperature for 75 minutes and then further stirred at 50°C for 2 hours. The reaction mixture was filtered to separate the 20% palladium hydroxide/carbon and the residue was then washed with hot methanol. The washings were combined with the filtrate and the solvent was distilled off under reduced pressure. To the residue, a 4 N hydrochloric acid 1,4-dioxane solution (10 mL) was added. The resulting mixture was stirred at room temperature for 1 hour and ethyl acetate was then added to the mixture to precipitate a crude product, which was then filtered and washed with ethyl acetate. The solvent was distilled off under reduced pressure to yield the title compound (46.6 mg).
Rf=0.8 (4:1 chloroform/methanol (free form));
¹H-NMR (DMSO-d₆): 10.92 (1H, br), 9.87 (1H, s), 9.33 (1H, s), 8.80-9.27 (2H, br), 7.81 (1H, d, J=8.6), 7.63 (1H, d, J=8.6), 7.36 (1H, t, J=7.9), 7.31 (1H, s), 7.15 (2H, t, J=7.6), 6.94 (1H, d, J=2.0), 6.79 (1H, d, J=2.3), 6.76 (1H, dd, J=8.6, 2.0), 6.59 (1H, dd, J=8.6, 2.3), 6.27 (1H, d, J=3.3), 5.01 (1H, dd, J=10.2, 3.3), 4.36 (2H, m), 3.46 (2H, m), 3.00-3.34 (2H, m), 3.00 (3H, s);
Mass (m/e): 456 (MH⁺).

### [Examples 3 to 5]

Reactions similar to Example 2 were carried out to prepare compounds having the combinations of R¹, R², R³ and W specified in Table 1 included within the general formula (I).

### Example 3: Preparation of (R)-N-[2-chloro-5-[2-[2-(7-hydroxy-9H-carbazol-2-yloxy)ethylamino]-1-hydroxyethyl]phenyl]methanesulfonamide hydrochloride

Rf=0.06 (90:9:1 chloroform/methanol/28% aqueous ammonia (free form));
¹H-NMR (DMSO-d₆): 10.90 (1H, br), 9.55 (1H, s), 9.33 (1H, br), 9.12 (1H, br), 8.98 (1H, br), 7.82 (1H, d, J=8.2), 7.74 (1H, d, J=8.5), 7.56 (1H, d, J=8.2), 7.53 (1H, s), 7.31 (1H, d, J=8.2), 6.94 (1H, s), 6.79 (1H, s), 6.77 (1H, d, J=8.8), 6.59 (1H, d, J=8.2), 6.36 (1H, br), 5.04 (1H, d, J=9.6), 4.36 (2H, m), 3.40-3.50 (2H,m), 3.00-3.40 (2H, m), 3.06 (3H, s);
Mass (m/e): 490 (MH⁺).

### [Intermediate 4]

### Preparation of 7-fluoro-2-hydroxy-9H-carbazole

### A. Preparation of 5-fluoro-2-(4-methoxyphenyl)nitrobenzene

2-Bromo-5-fluoronitrobenzene (5.0 g) was dissolved in toluene (45 mL). Tetrakistriphenylphosphine palladium(0) (787 mg) and an aqueous potassium carbonate solution (22.5 mL) which had been adjusted to 2 M were added. 4-Methoxyphenylboronic acid (3.8 g) and ethanol (20 mL) were added and the resulting mixture was stirred at 90°C for 23 hours. The reaction liquid was cooled to room temperature and further cooled with ice. An aqueous 30% hydrogen peroxide solution (1.25 mL) was gradually added dropwise. The resulting mixture was brought back to room temperature and then stirred for 1 hour. The mixture was extracted with diisopropyl ether, and the organic layer was washed with saturated brine and dried. The solvent was then removed under reduced pressure. The residue was purified by silica gel column chromatography (49:1 hexane/ethyl acetate) to yield the title compound (4.67 g).
Rf=0.50 (3:1 hexane/ethyl acetate);
¹H-NMR (DMSO-d₆): 7.96 (1H, dd, J=8.5, 2.5), 7.57-7.68 (2H, m), 7.24-7.28 (2H, m), 6.99-7.04 (2H, m), 3.80 (3H, s);
Mass (m/e): 248 (MH⁺).

### B. Preparation of 7-fluoro-2-methoxy-9H-carbazole

A compound (4.67 g; prepared according to the procedure of the step A of Intermediate 4) was added to triethyl phosphite (10 mL) and the resulting mixture was stirred at 160°C for 7.5 hours. After the reaction was completed, the reaction mixture was cooled to room temperature and further cooled with ice. An aqueous 7.5% hydrogen peroxide solution (40 mL) was gradually added dropwise. The precipitated crystal was then collected by filtration and dried in vacuo to yield the title compound (3.49 g).
Rf=0.29 (3:1 hexane/ethyl acetate);
¹H-NMR (DMSO-d₆): 11.22 (1H, br), 7.99 (1H, d, J=8.1), 7.94 (1H, d, J=8.1), 7.18-7.22 (1H, m), 6.90-6.97 (2H, m), 6.76-6.80 (1H, m), 3.83 (3H, s);
Mass (m/e): 216 (MH⁺).

### C. Preparation of 7-fluoro-2-hydroxy-9H-carbazole

A compound (1.93 g; prepared according to the procedure of the step B of Intermediate 4) was reacted with pyridine hydrochloride (10.4 g) under reaction conditions similar to those in the step F of Intermediate 1 to yield the title compound (1.36 g).
Rf=0.80 (9:1 chloroform/methanol);
¹H-NMR (DMSO-d₆): 11.05 (1H, br), 9.40 (1H, s), 7.90 (1H, dd, J=8.5, 5.6), 7.82 (1H, d, J=8.5), 7.13 (1H, dd, J=10.2, 2.2), 6.86-6.93 (1H, m), 6.81 (1H, d, J=2.2), 6.63 (1H, dd, J=8.5, 2.2);
Mass (m/e): 202 (MH⁺).

### [Example 6]

### Preparation of (R)-N-[3-[2-[2-(7-fluoro-9H-carbazol-2-yloxy)ethylamino]-1-hydroxyethyl]phenyl]methanesulfonamide hydrochloride

### A. Preparation of (R)-N-benzyl-N-[3-[2-[N'-benzyl-2-(7-fluoro-9H-carbazol-2-yloxy)ethylamino]-1-hydroxyethyl]phenyl]methanesulfonamide

A compound (1.40 g; prepared according to the procedure of the step A of Example 2) was dissolved in methylene chloride (25 mL). Carbon tetrabromide (1.24 g) and triphenylphosphine (1.24 g) were added and the resulting mixture was reacted under reaction conditions similar to those in the step B of Example 2. The thus obtained crude product was not purified and then was dissolved in THF (25 mL). A compound (500 mg; prepared according to the procedure of the step C of Intermediate 4) and an aqueous 1 N sodium hydroxide solution were added, and the resulting mixture was reacted under reaction conditions similar to those in the step C of Example 2 to yield the title compound (1.11 g).
Rf=0.49 (9:1 chloroform/methanol);
¹H-NMR (DMSO-d₆): 11.19 (1H, br), 7.98 (1H, dd, J=8.5, 5.8), 7.93 (1H, d, J=8.5), 7.15-7.35 (15H, m), 6.90-6.97 (2H, m), 6.73 (1H, dd, J=8.5, 2.3), 5.13 (1H, d, J=3.6), 4.81 (2H, s), 4.67 (1H, br), 3.96-3.99 (2H, m), 3.75 (2H, s), 3.04 (3H, s), 2.86-2.93 (2H, m), 2.65-2.70 (2H, m);
Mass (m/e): 639 (MH⁺).

### B. Preparation of (R)-N-[3-[2-[2-(7-fluoro-9H-carbazol-2-yloxy)-ethylamino]-1-hydroxyethyl]phenyl]methanesulfonamide hydrochloride

A compound (300 mg; prepared according to the procedure of the step A of Example 6) was dissolved in ethanol (20 mL) under an argon atmosphere, and 20% palladium hydroxide/carbon (60 mg) was then added. After the argon stream was replaced with hydrogen gas, the resulting mixture was reacted under reaction conditions similar to those in the step D of Example 2 to yield the title compound (228 mg).
Rf=0.13 (9:1 chloroform/methanol (free form));
¹H-NMR (DMSO-d₆): 11.33 (1H, br), 9.86 (1H, s), 8.90-9.20 (1H, br) , 8.00 (2H, d, J=8.2), 7.31-7.39 (2H, m), 7.23 (1H, dd, J=10.0, 2.3), 7.15 (2H, t, J=7.1), 7.04 (1H, d, J=1.9), 6.92-6.99 (1H, m), 6.87 (1H, s), 6.27 (1H, br), 5.00 (1H, br), 4.39 (2H, s), 3.40-3.50 (2H, m), 3.25-3.35 (1H, m), 3.03-3.15 (1H, m), 3.00 (3H, s);
Mass (m/e): 459 (MH⁺).

### [Intermediate 5]

### Preparation of 2-hydroxy-7-methoxy-9H-carbazole

### A. Preparation of 5-benzyloxy-2-bromonitrobenzene

2-Bromo-5-hydroxynitrobenzene (1.0 g) was dissolved in acetone (50 mL). Potassium carbonate (3.5 g) and benzyl bromide (1.2 mL) were added, and the resulting mixture was stirred at room temperature for 3 hours. After the reaction was completed, water (100 mL) was added and the mixture was extracted with ethyl acetate. The organic layer was washed with saturated brine and dried. The solvent was then distilled off under reduced pressure. The residue was purified by silica gel column chromatography (19:1 hexane/ethyl acetate) to yield the title compound (1.42 g).
Rf=0.38 (9:1 hexane/ethyl acetate);
¹H-NMR (DMSO-d₆): 7.79 (1H, d, J=9.1), 7.74 (1H, d, J=3.0), 7.35-7.48 (5H, m), 7.28 (1H, dd, J=9.1, 3.0), 5.20 (2H, s);
Mass (m/e): 309 (MH⁺).

### B. Preparation of 5-benzyloxy-2-(4-methoxyphenyl)nitrobenzene

A compound (1.0 g; prepared according to the procedure of the step A of Intermediate 5) was dissolved in toluene (20 mL). Tetrakistriphenylphosphine palladium(0) (115 mg) and an aqueous potassium carbonate solution (3.3 mL) which had been adjusted to 2 M were added. 4-Methoxyphenylboronic acid (1.0 g) and ethanol (5 mL) were added and the resulting mixture was reacted under reaction conditions similar to those in the step A of Intermediate 4. The thus obtained crude product was purified by silica gel column chromatography (9:1 hexane/ethyl acetate) to yield the title compound (250 mg).
Rf=0.49 (3:1 hexane/ethyl acetate);
¹H-NMR (DMSO-d₆): 7.59 (1H, d, J=2.5), 7.30-7.50 (7H, m), 7.22 (2H, d, J=8.8), 6.99 (2H, d, J=8.8), 5.24 (2H, s), 3.79 (3H, s);
Mass (m/e): 336 (MH⁺).

### C. Preparation of 2-benzyloxy-7-methoxy-9H-carbazole

A compound (250 mg; prepared according to the procedure of the step B of Intermediate 5) was reacted with triethyl phosphite (3 mL) under reaction conditions similar to those in the step B of Intermediate 4 to yield the title compound (142 mg).
Rf=0.24 (3:1 hexane/ethyl acetate);
¹H-NMR (DMSO-d₆): 10.98 (1H, br), 7.85 (2H, dd, J=8.7, 2.1), 7.33-7.51 (5H, m), 7.00 (1H, d, J=2.2), 6.92 (1H, d, J=2.2), 6.81 (1H, dd, J=8.5, 2.2), 6.72 (1H, dd, J=8.5, 2.2), 5.17 (2H, s), 3.81 (3H,s);
Mass (m/e): 304 (MH⁺).

### D. Preparation of 2-hydroxy-7-methoxy-9H-carbazole

A compound (142 mg; prepared according to the procedure of the step C of Intermediate 5) was dissolved in a mixed solvent of THF (25 mL) and ethanol (15 mL) under an argon atmosphere, and 20% palladium hydroxide/carbon (70 mg) was then added. After the argon stream was replaced with hydrogen gas, the resulting mixture was stirred at room temperature for 2 hours. The reaction mixture was filtered to separate the 20% palladium hydroxide/carbon and the residue was then washed with THF. The washings were combined with the filtrate and the solvent was distilled off under reduced pressure to yield the title compound (100 mg).
Rf=0.12 (3:1 hexane/ethyl acetate);
¹H-NMR (DMSO-d₆): 10.79 (1H, br), 9.23 (1H, s), 7.77 (1H, d, J=8.5), 7.72 (1H, d, J=8.5), 6.87 (1H, d, J=2.2), 6.76 (1H, d, J=1.7), 6.68 (1H, dd, J=8.5, 2.2), 6.57 (1H, dd, J=8.5, 2.2), 3.80 (3H, s);
Mass (m/e): 214 (MH⁺).

### [Example 7]

### Preparation of (R)-N-[3-[2-[2-(7-methoxy-9H-carbazol-2-yloxy)ethylamino]-1-hydroxyethyl]phenyl]methanesulfonamide hydrochloride

### A. Preparation of (R)-N-benzyl-N-[3-[2-[N'-benzyl-2-(7-methoxy-9H-carbazol-2-yloxy)ethylamino]-1-hydroxyethyl]phenyl]methanesulfonamide

A compound (43 mg; prepared according to the procedure of the step D of Intermediate 5) was dissolved in N,N-dimethylacetamide (2 mL), and potassium carbonate (83 mg) was then added. A solution of a compound (0.19 g; prepared according to the procedure of the step B of Example 2) in N,N-dimethylacetamide (2 mL) was added, and the resulting mixture was stirred at room temperature for 3 days. Water (25 mL) was added and the reaction liquid was extracted with ethyl acetate three times. The organic layer was washed with saturated brine and dried. After the solvent was distilled off, the residue was purified by silica gel column chromatography (19:1 chloroform/methanol) and then repurified by silica gel column chromatography (1:1 hexane/ethyl acetate) to yield the title compound (118 mg).
Rf=0.61 (9:1 chloroform/methanol);
¹H-NMR (CDCl₃): 8.10 (1H, s), 7.84 (1H, d, J=1.7), 7.82 (1H, d, J=1.7), 7.18-7.36 (13H, m), 6.92 (1H, d, J=2.2), 6.91 (1H, d, J=2.8), 6.84 (1H, d, J=2.2), 6.83 (1H, dd, J=3.0, 2.2), 6.79 (1H, d, J=2.2), 4.67 (1H, dd, J=10.2, 3.3), 4.10 (2H, m), 3.96 (1H, d, J=14.0), 3.89 (3H, s), 3.70 (1H, d, J=13.7), 2.96-3.16 (2H, m), 2.90 (3H, s), 2.58-2.86 (2H, m);
Mass (m/e): 650 (MH⁺).

### B. Preparation of (R)-N-[3-[2-[2-(7-methoxy-9H-carbazol-2-yloxy)-ethylamino]-1-hydroxyethyl]phenyl]methanesulfonamide hydrochloride

A compound (100 mg; prepared according to the procedure of the step A of Example 7) was dissolved in a mixed solvent of methanol (3 mL) and acetic acid (0.1 mL) under an argon atmosphere, and 20% palladium hydroxide/carbon (100 mg) was then added. After replacing the argon stream with hydrogen gas, the mixture was stirred at 55°C for 2 hours. The reaction mixture was filtered to separate the 20% palladium hydroxide/carbon and the residue was then washed with hot methanol. The washings were combined with the filtrate. After a 0.5 N hydrochloric acid ethanol solution (0.4 mL) was added, the solvent was distilled off under reduced pressure. The residue was dried under reduced pressure to yield the title compound (68 mg).
Rf=0.12 (90:9:1 chloroform/methanol/28% aqueous ammonia (free form));
¹H-NMR (DMSO-d₆): 11.07 (1H, s), 9.85 (1H, s), 9.30-9.60 (2H, br), 7.89 (1H, d, J=6.9), 7.86 (1H, d, J=6.6), 7.37 (1H, d, J=7.7), 7.31 (1H, s), 7.10-7.30 (2H, m), 7.00 (1H, d, J=2.2), 6.94 (1H, d, J=2.2), 6.79 (1H, dd, J=8.5, 2.2), 6.72 (1H, dd, J=8.5, 2.2), 6.27 (1H, s), 5.00 (1H, m), 4.37 (2H, m), 3.47 (2H, m), 3.0-3.4 (2H, m), 3.00 (3H, s);
Mass (m/e): 470 (MH⁺).

### [Intermediate 6]

### Preparation of 7-acetamido-2-hydroxy-9H-carbazole

### A. Preparation of 2-(4-aminophenyl)-5-benzyloxynitrobenzene

A compound (1.42 g; prepared according to the procedure of the step A of Intermediate 5) was dissolved in toluene (20 mL). Tetrakistriphenylphosphine palladium(0) (580 mg) and an aqueous potassium carbonate solution (5 mL) which had been adjusted to 2 M were added. 4-(4,4,5,5-Tetramethyl-1,3,2-dioxaboran-2-yl)aniline (1.41 g) and ethanol (5 mL) were added, and the resulting mixture was reacted under reaction conditions similar to those in the step A of Intermediate 4 to yield the title compound (1.37 g).
Rf=0.63 (1:1 hexane/ethyl acetate);
¹H-NMR (DMSO-d₆): 7.32-7.51 (8H, m), 6.93 (2H, d, J=8.4), 6.59 (2H, d, J=8.4), 5.29 (2H, s), 5.21 (2H, s);
Mass (m/e): 321 (MH⁺).

### B. Preparation of 2-(4-acetamidophenyl)-5-benzyloxynitrobenzene

A compound (1.37 g; prepared according to the procedure of the step A of Intermediate 6) was dissolved in methylene chloride (20 mL), and triethylamine (3 mL) and N,N-dimethylaminopyridine (52 mg) were then added. To the resulting mixture, acetic anhydride (1 mL) was slowly added dropwise with ice cooling. The mixture was gradually brought back to room temperature with stirring over 5 hours. Acetic anhydride (0.5 mL) was further added and the resulting mixture was stirred at room temperature for 25 hours. After the reaction was completed, the solvent was distilled off under reduced pressure. The residue was purified by silica gel column chromatography (1:1 hexane/ethyl acetate) to yield the title compound (947 mg).
Rf=0.55 (ethyl acetate);
¹H-NMR (DMSO-d₆): 10.05 (1H, s), 7.62 (2H, d, J=8.4), 7.36-7.50 (8H, m), 7.21 (2H, d, J=8.4), 5.24 (2H, s), 2.09 (3H, s);
Mass (m/e): 363 (MH⁺).

### C. Preparation of 7-acetamido-2-benzyloxy-9H-carbazole

A compound (947 mg; prepared according to the procedure of the step B of Intermediate 6) and triethyl phosphite (7 mL) were reacted as in the step B of Intermediate 4 to yield the title compound (270 mg).
Rf=0.42 (ethyl acetate);
¹H-NMR (DMSO-d₆): 11.04 (1H, br), 9.97 (1H, s), 7.97 (1H, s), 7.86 (2H, dd, J=8.4, 5.4), 7.33-7.51 (5H, m), 7.13 (1H, dd, J=8.4, 1.8), 6.99 (1H, d, J=2.2), 6.82 (1H, dd, J=8.5, 2.2), 5.18 (2H, s), 2.07 (3H, s);
Mass (m/e): 331 (MH⁺).

### D. Preparation of 7-acetamido-2-hydroxy-9H-carbazole

A compound (270 mg; prepared according to the procedure of the step C of Intermediate 6) was dissolved in a mixed solvent of THF (25 mL) and ethanol (15 mL) under an argon atmosphere, and then reacted under reaction conditions similar to those in the step D of Intermediate 5 to yield the title compound (200 mg).
Rf=0.12 (3:1 hexane/ethyl acetate);
¹H-NMR (DMSO-d₆) : 10.85 (1H, br), 9.93 (1H, s), 9.29 (1H, s), 7.91 (1H, s), 7.78 (1H, d, J=8.4), 7.74 (1H, d, J=8.4), 7.10 (1H, d, J=8.4), 6.75 (1H, s), 6.58 (1H, dd, J=8.4, 2.2), 2.06 (3H,s);
Mass (m/e): 241 (MH⁺).

### [Example 8]

### Preparation of (R)-N-[3-[2-[2-(7-acetamido-9H-carbazol-2-yloxy)-ethylamino]-1-hydroxyethyl]phenyl]methanesulfonamide hydrochloride

### A. Preparation of (R)-N-benzyl-N-[3-[2-[N'-benzyl-2-(7-acetamide-9H-carbazol-2-yloxy)ethylamino]-1-hydroxyethyl]phenyl]methanesulfonamide

A compound (120 mg; prepared according to the procedure of the step D of Intermediate 6) was dissolved in N,N-dimethylacetamide (5 mL), and potassium carbonate (207 mg) was then added. To this reaction liquid, a solution of a compound (0.55 g; prepared according to the procedure of the step B of Example 2) in N,N-dimethylacetamide (5 mL) was added. The resulting mixture was stirred at room temperature for four days. The reaction liquid was extracted with ethyl acetate four times and the organic layer was then washed with saturated brine and dried. After the solvent was distilled off under reduced pressure, the residue was purified by silica gel column chromatography (40:1 chloroform/methanol) and then repurified by silica gel column chromatography (1:1 to 0:1 hexane/ethyl acetate) to yield the title compound (170mg).
Rf=0.38 (9:1 chloroform/methanol);
¹H-NMR (CDCl₃): 8.27 (1H, s), 8.02 (1H, d, J=1.7), 7.85 (1H, s), 7.82 (1H, s), 7.41 (1H, s), 7.18-7.34 (13H, m), 7.09-7.13 (1H, m), 6.94 (1H, dd, J=8.2, 1.9), 6.89 (1H, d, J=2.2), 6.81 (1H, dd, J=8.5, 2.2), 4.78 (2H, d, J=1.1), 4.66 (1H, dd, J=10.2, 3.3), 4.08 (2H, m), 3.95 (1H, d, J=13.5), 3.69 (1H, d, J=13.7), 2.95-3.15 (2H, m), 2.89 (3H, s), 2.82 (1H, dd, J=12.9, 3.3), 2.60 (1H, dd, J=12.9, 10.4), 2.21 (3H, s);
Mass (m/e): 677(MH⁺).

### B. Preparation of (R)-N-[3-[2-[2-(7-acetamido-9H-carbazol-2-yloxy)ethylamino]-1-hydroxyethyl]phenyl]methanesulfonamide hydrochloride

A compound (150 mg; prepared according to the procedure of the step A of Example 8) was dissolved in a mixed solvent of methanol (4.5 mL) and acetic acid (0.15 mL) under an argon atmosphere, and 20% palladium hydroxide/carbon (150 mg) was then added. After the argon stream was replaced with hydrogen gas, the resulting mixture was stirred at room temperature for 4 hours and then further stirred at 50°C for 2 hours. The reaction mixture was filtered to separate the 20% palladium hydroxide/carbon and the residue was then washed with hot methanol. The washings were combined with the filtrate. After a 0.5 N hydrochloric acid ethanol solution (0.5 mL) was added, the solvent was distilled off under reduced pressure. The residue was dried in vacuo to yield the title compound (88 mg).
Rf=0.26 (4:1 chloroform/methanol (free form));
¹H-NMR (DMSO-d₆): 11.13 (1H, br), 10.03 (1H, s), 9.86 (1H, s), 9.11 (1H, br), 8.93 (1H, br), 8.01 (1H, d, J=1.4), 7.90 (1H, d, J=8.5), 7.87 (1H, d, J=9.9), 7.36 (1H, d, J=8.0), 7.31 (1H, s), 7.1-7.2 (3H, m), 6.98 (1H, d, J=2.2), 6.80 (1H, dd, J=8.5, 2.2), 6.26 (1H, br), 5.00 (1H, d, J=9.6), 4.38 (2H, m), 3.47 (2H, m), 3.20-3.40 (1H, m), 3.00-3.20 (1H, m), 3.00 (3H, s), 2.08 (3H, 5);
Mass (m/e): 497(MH⁺).

### [Example 9]

### Preparation of (R)-N-[3-[2-[2-(7-amino-9H-carbazol-2-yloxy)ethylamino]-1-hydroxyethyl]phenyl]methanesulfonamide dihydrochloride

A compound (45 mg; prepared according to the procedure of the step B of Example 8) was dissolved in a mixed solvent of methanol (5 mL) and aqueous 1 N hydrochloric acid (5 mL) under an argon atmosphere. The resulting reaction liquid was stirred overnight at 75°C. The reaction liquid was concentrated under reduced pressure and the precipitated crystal was collected by filtration. The crystal was dried under reduced pressure to yield the title compound (19 mg).
Rf=0.24 (4:1 chloroform/methanol (free form));
¹H-NMR (DMSO-d₆): 11.49 (1H, br), 9.90-10.30 (3H, br), 9.86 (1H, s), 9.22 (1H, br), 8.98 (1H, br), 8.07 (1H, d, J=8.5), 8.03 (1H, d, J=8.8), 7.45 (1H, s), 7.31 (1H, s), 7.1-7.2 (3H, m), 7.00-7.10 (2H, m), 6.87 (1H, dd, J=8.8, 2.2), 6.27 (1H, br), 5.01 (1H, d, J=10.2), 4.41 (2H, m), 3.20-3.50 (3H, m), 3.00-3.20 (1H, m), 3.00 (3H, s);
Mass (m/e): 455 (MH⁺).

### [Intermediate 7]

### Preparation of 2-hydroxy-7-pivaloyloxy-9H-carbazole

### A. Preparation of 2-bromo-5-pivaloyloxynitrobenzene

2-Bromo-5-hydroxynitrobenzene (2.8 g) was added to pyridine (50 mL) and the resulting mixture was cooled with ice. Pivaloyl chloride (5.2 mL) was added dropwise and the mixture was slowly brought back to room temperature with stirring over 3 hours. The reaction was completed, the solvent was distilled off under reduced pressure. The residue was purified by silica gel column chromatography (1:1 hexane/ethyl acetate) to yield the title compound (4.4 g).
Rf=0.72 (1:1 hexane/ethyl acetate);
¹H-NMR (DMSO-d₆): 7.99 (1H, d, J=2.8), 7.97 (1H, d, J=8.8), 7.46 (1H, dd, J=8.8, 2.8), 1.32 (9H, s);
Mass (m/e): 303 (MH⁺).

### B. Preparation of 2-(4-benzyloxyphenyl)-5-pivaloyloxynitrobenzene

A compound (500 mg; prepared according to the procedure of the step A of Intermediate 7) was dissolved in toluene (20 mL). To the resulting reaction liquid, tetrakistriphenylphosphine palladium(0) (60 mg) and an aqueous sodium carbonate solution (2 mL) which had been adjusted to 2 M were added. 4-Benzyloxyphenylboronic acid (821 mg) and ethanol (5 mL) were added and the resulting mixture was reacted under reaction conditions similar to those in the step A of Intermediate 4 to yield the title compound (710 mg).
Rf=0.59 (9:1 hexane/ethyl acetate);
¹H-NMR (DMSO-d₆): 7.85 (1H, d, J=2.5), 7.35-7.60 (7H, m), 7.29 (2H, dd, J=6.6, 2.2), 7.10 (2H, dd, J=6.6, 2.2), 5.15 (2H, s), 1.34 (9H, s);
Mass (m/e): 406 (MH⁺).

### C. Preparation of 2-benzyloxy-7-pivaloyloxy-9H-carbazole

A compound (710 mg; prepared according to the procedure of the step B of Intermediate 7) and triethyl phosphite (1 mL) were reacted under reaction conditions similar to those in the step B of Intermediate 4 to yield the title compound (261 mg).
Rf=0.34 (3:1 hexane/ethyl acetate);
¹H-NMR (DMSO-d₆): 11.18 (1H, br), 7.89 (2H, dd, J=8.5, 3.6), 7.34-7.52 (5H, m), 7.09 (2H, dd, J=14.4, 2.2), 6.87 (1H, dd, J=8.5, 2.2), 6.81 (1H, dd, J=8.5, 2.2), 5.20 (2H, s), 1.33 (9H, s);
Mass (m/e): 374 (MH⁺).

### D. Preparation of 2-hydroxy-7-pivaloyloxy-9H-carbazole

A compound (261 mg; prepared according to the procedure of the step C of Intermediate 7) was dissolved in a mixed solvent of THF (5 mL) and ethanol (10 mL) under an argon atmosphere and reacted under reaction conditions similar to those in the step D of Intermediate 5 to yield the title compound (213 mg).
Rf=0.10 (3:1 hexane/ethyl acetate);
¹H-NMR (DMSO-d₆): 11.00 (1H, br), 9.40 (1H, s), 7.90 (1H, d, J=8.2), 7.84 (1H, d, J=8.2), 7.06 (1H, d, J=2.2), 6.82 (1H, d, J=2.2), 6.77 (1H, dd, J=8.2, 2.2), 6.63 (1H, dd, J=8.2, 2.2), 1.33 (9H, s);
Mass (m/e): 284 (MH⁺).

### [Intermediate 8]

### Preparation of 2-hydroxy-7-bromo-9H-carbazole

### A. Preparation of 5-benzyloxy-2-(4-bromophenyl)nitrobenzene

A compound (1.0 g; prepared according to the procedure of the step A of Intermediate 5) was dissolved in toluene (40 mL). To the resulting reaction liquid, [1,1'-bis(diphenylphosphino)-ferrocene] palladium(II) (73 mg) and an aqueous sodium carbonate solution (3.3 mL) which had been adjusted to 2 M were added. 4-Bromophenylboronic acid (3.3 g) and ethanol (5 mL) were added and the resulting mixture was reacted under reaction conditions similar to those in the step A of Intermediate 4 to yield the title compound (1.2 g).
Rf=0.52 (3:1 hexane/ethyl acetate);
¹H-NMR (DMSO-d₆): 7.83-7.39 (12H, m), 5.27 (2H, s);
Mass (m/e): 385 (MH⁺).

### B. Preparation of 2-benzyloxy-7-bromo-9H-carbazole

A compound (1.2 g; prepared according to the procedure of the step A of Intermediate 8) and triethyl phosphite (3.5 mL) were reacted under reaction conditions similar to those in the step B of Intermediate 4 to yield the title compound (113 mg).
Rf=0.51 (3:1 hexane/ethyl acetate);
¹H-NMR (DMSO-d₆): 11.22 (1H, br), 8.07 (1H, d, J=8.2), 8.01 (1H, d, J=8.2), 7.30-7.85 (7H, m), 7.07 (1H, d, J=2.2), 6.88 (1H, dd, J=8.2, 2.2), 5.21 (2H, s);
Mass (m/e): 353 (MH⁺).

### C. Preparation of 2-hydroxy-7-bromo-9H-carbazole

A compound (10 mg; prepared according to the procedure of the step B of Intermediate 8) was dissolved in a mixed solvent of THF (5 mL) and ethanol (2 mL) under an argon atmosphere, and 20% palladium hydroxide/carbon (5 mg) was then added. After the argon stream was replaced with hydrogen gas, the resulting mixture was reacted under reaction conditions similar to those in the step D of Intermediate 5 to yield the title compound (6 mg).
Rf=0.13 (3:1 hexane/ethyl acetate);
¹H-NMR (DMSO-d₆): 11.04 (1H, br), 9.38 (1H, s), 8.00 (1H, d, J=8.5), 7.88 (1H, d, J=8.5), 7.81 (1H, d, J=8.5), 7.60-7.75 (1H, m), 6.83 (1H, d, J=2.2), 6.64 (1H, dd, J=8.5, 2.2);
Mass (m/e): 263 (MH⁺).

### [Intermediate 9]

### Preparation of 7-cyano-2-hydroxy-9H-carbazole

### A. Preparation of 2-benzyloxy-7-cyano-9H-carbazole

The compound (734 mg) prepared in the step B of Intermediate 8 was dissolved in dimethylformamide. Copper cyanide (606 mg) was added and the resulting mixture was reacted at 160°C for 22.5 hours. The reaction liquid was cooled to room temperature and added to ice water (100 mL). The precipitated crystal was collected by filtration and suspended in water (60 mL). Ethylenediamine (5 mL) and ethyl acetate (100 mL) were added and the resulting mixture was stirred for 30 minutes. The resulting solution was extracted with ethyl acetate and the organic layer was washed with an aqueous sodium cyanide solution (1 mol/L), water and brine, and dried. The solvent was then distilled off under reduced pressure and the residue was purified by preparative TLC (3:1 hexane/ethyl acetate) to yield the title compound (140 mg).
Rf=0.30 (3:1 hexane/ethyl acetate);
¹H-NMR (DMSO-d₆): 11.60 (1H, br), 8.19 (1H, d, J=8.5), 8.12 (1H, d, J=8.5), 7.88-7.98 (2H, m), 7.32-7.52 (5H, m), 7.13 (1H, d, J=2.2), 6.95 (1H, dd, J=8.5, 2.2), 5.23 (2H, s) ;
Mass (m/e): 299 (MH⁺).

### B. Preparation of 7-cyano-2-hydroxy-9H-carbazole

The compound (10 mg) prepared in the step A of Intermediate 9 was dissolved in a mixed solvent of ethanol (1 mL) and THF (1 mL). Palladium hydroxide/carbon (5 mg) was added and the resulting mixture was reacted under reaction conditions similar to those in the step D of Intermediate 5 to yield the title compound (6 mg).
Rf=0.43 (1:1 hexane/ethyl acetate);
¹H-NMR (DMSO-d₆): 11.41 (1H, br), 9.76 (1H, br), 8.12 (1H, d, J=8.0), 7.99 (1H, d, J=8.5), 7.83 (1H, br), 7.43-7.47 (1H, m), 6.87 (1H, br), 6.70-6.74 (1H, m);
Mass (m/e): 207 (MH⁻).

### [Examples 10 to 30]

Each of the compounds having a combination of R¹, R², R³ and W specified in Table 2 and encompassed within the compounds of the general formula (I) was prepared by a reaction similar to Example 2 using intermediates 1 to 9.

**[Table 1]**

| Example | R¹ | R² | R³ | W |
|---|---|---|---|---|
| 3 | Cl | CH₃ | OH | NH |
| 4 | Br | CH₃ | OH | NH |
| 5 | OH | CH₃ | OH | NH |

**[Table 2]**

| Example | R¹ | R² | R³ | W |
|---|---|---|---|---|
| 10 | H | CH₃ | OCOC(CH₃)₃ | NH |
| 11 | H | CH₃ | CN | NH |
| 12 | Cl | CH₃ | F | NH |
| 13 | Cl | CH₃ | NHAc | NH |
| 14 | Cl | CH₃ | NH₂ | NH |
| 15 | Cl | CH₃ | OCOC(CH₃)₃ | NH |
| 16 | Cl | CH₃ | Br | NH |
| 17 | Cl | CH₃ | OCH₃ | NH |
| 18 | Cl | CH₃ | CN | NH |
| 19 | Br | CH₃ | OCH₃ | NH |
| 20 | Br | CH₃ | NHAc | NH |
| 21 | Br | CH₃ | NH₂ | NH |
| 22 | Br | CH₃ | OCOC(CH₃)₃ | NH |
| 23 | Br | CH₃ | Br | NH |
| 24 | Br | CH₃ | CN | NH |
| 25 | Br | CH₃ | F | NH |
| 26 | OH | CH₃ | NHAc | NH |
| 27 | OH | CH₃ | NH₂ | NH |
| 28 | OH | CH₃ | F | NH |
| 29 | OH | CH₃ | OCH₃ | NH |
| 30 | OH | CH₃ | Br | NH |

### [Test Example 1]

### Human β3-agonist activities

Human β3-agonist activities were determined using CHO (Chinese hamster ovary) cells transfected with pcDNA3 (mfd. by Invitrogen) to which human β3 gene had been inserted. Human β3 fragment was first obtained from human adipose tissue cDNA (mfd. by Clonetech) by PCR using the primer of β3 (Krief, et al., J. Clin. Invest., 91, p. 344 (1993)). The human β3 fragment thus obtained was used as a probe to obtain the full length human β3 gene from a human genomic library (mfd. by Clonetech).

The above cells were cultured in a Ham F-12 medium supplemented with 10% fetal bovine serum (mfd. by Dainippon Pharmaceutical), 400 µg/mL geneticin (Gibco BRL), 100 U/mL penicillin and 100 µg/mL streptomycin. After placing these cells (5×10⁵) into a 6-well plate and culturing them for 24 hours, they were allowed to stand on a serum-free Ham F-12 medium for 2 hours. The compound was first dissolved in DMSO, repeatedly diluted by ten times with Ham F-12 supplemented with 1 mM isobutylmethylxanthine and 1 mM ascorbic acid to a final concentration of from 10⁻⁵ to 10⁻¹² M, and then added to the cells.

After the cells were cultured for 30 minutes, the medium was removed followed by addition of 0.5 mL of an aqueous 1 N sodium hydroxide solution. The medium was allowed to stand for 20 minutes and then 0.5 mL of an aqueous 1 N acetic acid solution was added to the medium. The medium was stirred and centrifuged followed by quantitating cAMP with cAMP EIA kit (mfd. by Cayman). Isoproterenol was purchased from RBI (Research Biochemicals International).

The compound of Example 2 showed a human β3-agonist activity in terms of ED₅₀ of 0.8 nM and intrinsic activity of 82% for Isoproterenol. Likewise, the compounds of Examples 3, 8, 9 and 10 showed human β3-agonist activities with 1.5 nM (75%), 0.35 nM (47%), 1.0 nM (102%) and 2.4 nM (112%), respectively. The compounds of the other Examples also showed human β3-agonist activities.

### [Test Example 2]

### Action on the heart

The heart was excised from a male guinea pig weighing 180-250 g to prepare a specimen of the right atrium. The specimen was set in an organ bath filled with a Krebs solution which had been aerated with a mixed gas of 5% CO₂/95% O₂. Each of the present compounds prepared in Examples was added to the Krebs solution. The automaticity was determined using a isometric transducer (NIHON KOHDEN TB-611T) connected to a polygraph (NIHON KOHDEN MR-6000). The compounds of the present invention showed higher ED₅₀ values for the automaticity as compared with ED₅₀ values for β3, and therefore are expected to have selective actions and hardly induce an increase of the heart rate. That is, the present compounds are expected to have few side effects.

### [Test Example 3]

### Pharmacological effect on a transgenic mouse expressing human β3

Since β3 is species specific (Strosberg, et al., Trends Pharmacol. Sci., 17, p. 373 (1996); Strosberg, et al., Annu. Rev. Pharmacol. Toxicol., 37, p. 421 (1997)), pharmacological tests using a transgenic mouse expressing human β3 are more effective than those using a normal mouse or rat. For example, Ito, et al., prepared a replacement mouse expressing human β3 in its brown fat by introducing human β3 gene into a mouse whose mouse β3 gene had been knocked out (Diabetes, 47, p. 1464 (1998)). Human β3 gene can be also expressed in a normal mouse instead of expression of human β3 in such a knocked out mouse. In addition, a pharmacological effect of a compound on the state of a disease can be also shown by using an obese and diabetic mouse with human β3 gene which is a progeny produced by crossing the transgenic mouse with a genetically obese and diabetic mouse such as ob/ob, db/db or agouti mouse. For example, human β3 gene can be expressed in a tissue which expresses mouse β3 gene by linking mouse β3 promoter to upstream of human β3 gene used in Test Example 1. The method of Hogan, et al. (A Laboratory Manual, 2nd Ed., Cold Spring Harbor Laboratory Press, Cold Spring Harbor, NY) can produce a transgenic mouse expressing human β3. The compounds of the present invention can be evaluated with this model to find the human β3 activities of the orally administered compounds.

### [Test Example 4]

### Toxicity test

Each of the present compounds prepared in Examples was orally administered to 6-week old male mice (CHARLES RIVER JAPAN) at 100 mg/kg, and none were found to be dead. This test showed a low toxicity of the present compounds.

All the publications, patents and patent applications cited in this specification are incorporated herein in their entities by reference.

### Industrial Utility

A compound of the present invention is a novel compound having an activity on human β3. The compound, which has a high human β3 activity, is believed to have clinically useful physical properties. Therefore, a compound of the present invention is useful as a pharmaceutical composition for treating and preventing β3-associated diseases, such as diabetes, obesity and hyperlipidemia.

## Claims

1. A compound of the general formula (I): 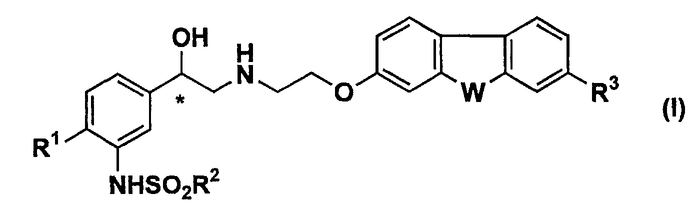 or a salt thereof,
wherein
R¹ represents a hydrogen atom, a halogen atom, or a hydroxyl group;
R² represents a lower alkyl group or a benzyl group;
R³ represents OR, a halogen atom, a trifluoromethyl group, a lower alkyl group, a lower acyl group, NR⁴R^{4'}, a nitro group, or a cyano group;
R represents a hydrogen atom, a lower alkyl group, a benzyl group, or an optionally substituted lower acyl group;
R⁴ and R^{4'} may be the same or different and represent a hydrogen atom, a lower alkyl group, a lower acyl group, a benzyl group, or SO₂R⁵;
R⁵ represents a lower alkyl group or a benzyl group;
W represents an oxygen atom, a secondary nitrogen atom (NH), or a sulfur atom; and
* represents an asymmetric carbon atom.

2. A compound as claimed in claim 1 having the general formula (I), wherein R¹ represents a hydrogen atom, a halogen atom, or a hydroxyl group; R² represents a lower alkyl group or a benzyl group; R³ represents OR, a halogen atom, a trifluoromethyl group, a lower alkyl group, NR⁴R^{4'}, a nitro group, or a cyano group; R represents a hydrogen atom, a lower alkyl group, or a benzyl group; R⁴ and R^{4'} may be the same or different and represent a hydrogen atom, a lower alkyl group, or a benzyl group; W represents an oxygen atom, a secondary nitrogen atom (NH), or a sulfur atom; and * represents an asymmetric carbon atom, or a salt thereof.

3. A compound as claimed in claim 1 or 2 having the general formula (I), wherein R³ represents OR, a halogen atom, a trifluoromethyl group, a lower alkyl group, NR⁴R^{4'}, or a cyano group, or a salt thereof.

4. A compound as claimed in any one of claims 1 to 3 having the general formula (I), wherein R¹ represents a hydrogen atom, a fluorine atom, a chlorine atom, a bromine atom, or a hydroxyl group; and R³ represents OR, or a salt thereof.

5. A compound as claimed in any one of claims 1 to 3 having the general formula (I), wherein R¹ represents a hydrogen atom, a fluorine atom, a chlorine atom, a bromine atom, or a hydroxyl group; and R³ represents NR⁴R^{4'}, or a salt thereof.

6. A compound as claimed in any one of claims 1 to 4 having the general formula (I), wherein R¹ represents a hydrogen atom, a fluorine atom, a chlorine atom, a bromine atom, or a hydroxyl group; and R³ represents a hydroxyl group, or a salt thereof.

7. A compound as claimed in any one of claims 4 to 6, which is selected from the group consisting of:
(R)-N-[3-[2-[2-(7-hydroxy-9H-carbazol-2-yloxy)ethylamino]-1-hydroxyethyl]phenyl]methanesulfonamide;
(S)-N-[3-[2-[2-(7-hydroxy-9H-carbazol-2-yloxy)ethylamino]-1-hydroxyethyl]phenyl]methanesulfonamide;
N-[3-[2-[2-(7-hydroxy-9H-carbazol-2-yloxy)ethylamino]-1-hydroxyethyl]phenyl]methanesulfonamide;
(R)-N-[5-[2-[2-(7-hydroxy-9H-carbazol-2-yloxy)ethylamino]-1-hydroxyethyl]-2-chlorophenyl]methanesulfonamide;
(S)-N-[5-[2-[2-(7-hydroxy-9H-carbazol-2-yloxy)ethylamino]-1-hydroxyethyl]-2-chlorophenyl]methanesulfonamide;
N-[5-[2-[2-(7-hydroxy-9H-carbazol-2-yloxy)ethylamino]-1-hydroxyethyl]-2-chlorophenyl]methanesulfonamide;
(R)-N-[5-[2-[2-(7-hydroxy-9H-carbazol-2-yloxy)ethylamino]-1-hydroxyethyl]-2-bromophenyl]methanesulfonamide;
(S)-N-[5-[2-[2-(7-hydroxy-9H-carbazol-2-yloxy)ethylamino]-1-hydroxyethyl]-2-bromophenyl]methanesulfonamide;
N-[5-[2- [2-(7-hydroxy-9H-carbazol-2-yloxy)ethylamino]-1-hydroxyethyl]-2-bromophenyl]methanesulfonamide;
(R)-N-[5-[2-[2-(7-hydroxy-9H-carbazol-2-yloxy)ethylamino]-1-hydroxyethyl]-2-hydroxyphenyl]methanesulfonamide;
(S)-N-[5-[2-[2-(7-hydroxy-9H-carbazol-2-yloxy)ethylamino]-1-hydroxyethyl]-2-hydroxyphenyl]methanesulfonamide;
N-[5-[2-[2-(7-hydroxy-9H-carbazol-2-yloxy)ethylamino]-1-hydroxyethyl]-2-hydroxyphenyl]methanesulfonamide;
(R)-N-[5-[2-[2-(7-amino-9H-carbazol-2-yloxy)ethylamino]-1-hydroxyethyl]phenyl]methanesulfonamide;
(R)-N-[5-[2-[2-(7-amino-9H-carbazol-2-yloxy)ethylamino]-1-hydroxyethyl]-2-chlorophenyl]methanesulfonamide;
(R)-N-[5-[2-[2-(7-acetylamino-9H-carbazol-2-yloxy)ethylamino]-1-hydroxyethyl]phenyl]methanesulfonamide; and
(R)-N-[5-[2-[2-(7-acetylamino-9H-carbazol-2-yloxy)ethylamino]-1-hydroxyethyl]-2-chlorophenyl]methanesulfonamide;
or a salt thereof.

8. A medicine comprising a compound according to claim 1 or a salt thereof as an active ingredient.

9. A medicine as claimed in claim 8, wherein the medicine is for treating or preventing diabetes, obesity or hyperlipidemia.

10. A process for the preparation of a compound of the general formula (I) or a salt thereof, comprising reacting a compound of the general formula (II): 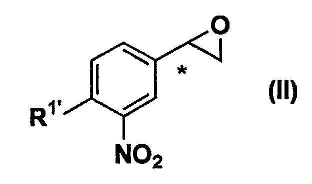 wherein R^{1'} represents a hydrogen atom, a halogen atom, or a protected hydroxyl group, and * represents an asymmetric carbon atom, with a compound of the general formula (III): 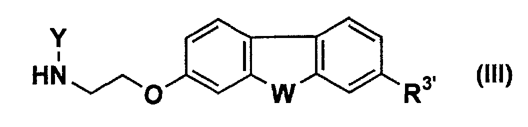 wherein W represents an oxygen atom, a secondary nitrogen atom (NH), or a sulfur atom; Y represents a hydrogen atom or an amine-protecting group; R^{3'} represents OR', a halogen atom, a trifluoromethyl group, a lower alkyl group, a lower acyl group, NR⁴R^{4'}, a nitro group, or a cyano group; R' represents a lower alkyl group, an optionally substituted lower acyl group, a benzyl group, or a hydroxyl-protecting group; R⁴ and R^{4'} may be the same or different and represent a hydrogen atom, a lower alkyl group, a lower acyl group, a benzyl group, an amine-protecting group, or SO₂R⁵; and R⁵ represents a lower alkyl group or a benzyl group, to give a compound of the general formula (IV): 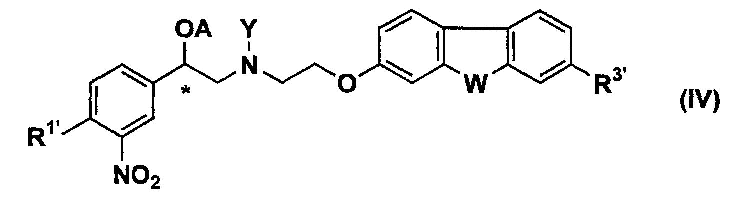 wherein A represents a hydrogen atom, and W, R^{1'}, Y, R^{3'} and * are each as defined above;
converting Y to an amine-protecting group when Y is a hydrogen atom;
reducing the compound of the general formula (IV) in which Y represents an amine-protecting group, to give a compound of the general formula (V): 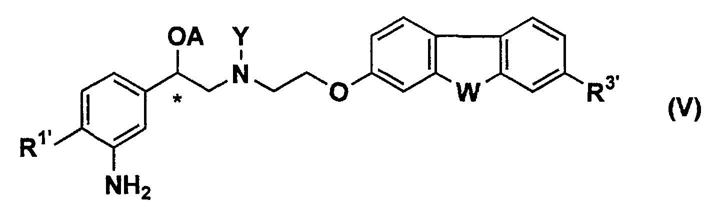 wherein Y represents an amine-protecting group, and W, R^{1'}, A, R^{3'} and * are each as defined above;
reacting the compound of the general formula (V) with a compound of the general formula (VI):
**XSO**_{**2**}**R**^{**2**} **(VI)**
wherein R² represents a lower alkyl group or a benzyl group, and X represents a leaving group, in the presence of an alkali to give a compound of the general formula (VII): 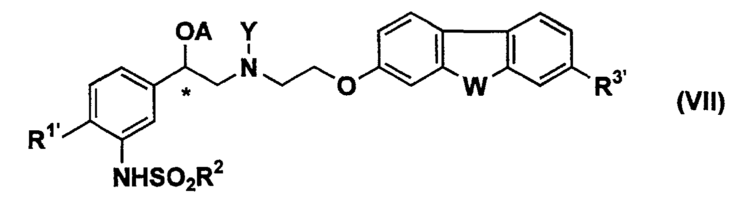 wherein A represents a hydrogen atom, and W, R^{1'}, Y, R², R^{3'} and * are each as defined above; and
when at least one of R^{1'}, R^{3'} and Y comprise a protecting group, simultaneously or sequentially removing the protecting group to give a compound of the general formula (I).

11. A process as claimed in claim 10, wherein the compound of the general formula (I) is a compound of the general formula (I) wherein R¹ represents a hydrogen atom, a halogen atom, or a hydroxyl group, R² represents a lower alkyl group or a benzyl group, R³ represents OR, a halogen atom, a trifluoromethyl group, a lower alkyl group, NR⁴R^{4'}, a nitro group, or a cyano group, R represents a hydrogen atom, a lower alkyl group, or a benzyl group, R⁴ and R^{4'} may be the same or different and represent a hydrogen atom, a lower alkyl group, or a benzyl group, W represents an oxygen atom, a secondary nitrogen atom (NH), or a sulfur atom, and * represents an asymmetric carbon atom, or a salt thereof; and wherein R^{3'} of the general formulae (III), (IV), (V) and (VII) represents OR', a halogen atom, a trifluoromethyl group, a lower alkyl group, NR⁴R^{4'}, a nitro group, or a cyano group wherein R' represents a lower alkyl group, a benzyl group, or a hydroxyl-protecting group, and R⁴ and R^{4'} may be the same or different and represent a hydrogen atom, a lower alkyl group, a benzyl group, or an amine-protecting group.

12. A compound of the general formula (III): 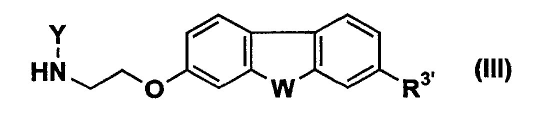 or a salt thereof,
wherein
W represents an oxygen atom, a secondary nitrogen atom (NH), or a sulfur atom;
Y represents a hydrogen atom or an amine-protecting group;
R^{3'} represents OR', a halogen atom, a trifluoromethyl group, a lower alkyl group, a lower acyl group, NR⁴R^{4'}, a nitro group, or a cyano group;
R' represents a lower alkyl group, an optionally substituted lower acyl group, a benzyl group, or a hydroxyl-protecting group;
R⁴ and R^{4'} may be the same or different and represent a hydrogen atom, a lower alkyl group, a lower acyl group, a benzyl group, an amine-protecting group, or SO₂R⁵; and
R⁵ represents a lower alkyl group or a benzyl group.

13. A compound as claimed in claim 12 having the general formula (III), wherein R^{3'} represents OR', a halogen atom, a trifluoromethyl group, a lower alkyl group, NR⁴R^{4'}, a nitro group, or a cyano group; R' represents a lower alkyl group, a benzyl group, or a hydroxyl-protecting group; R⁴ and R^{4'} may be the same or different and represent a hydrogen atom, a lower alkyl group, a benzyl group, or an amine-protecting group; and W represents an oxygen atom, a secondary nitrogen atom (NH), or a sulfur atom, or a salt thereof.

14. A compound as claimed in claim 12, which is selected from the group consisting of:
2-(7-fluoro-9H-carbazol-2-yloxy)ethylamine;
2-(7-chloro-9H-carbazol-2-yloxy)ethylamine;
2- (7-bromo-9H-carbazol-2-yloxy)ethylamine;
2-(7-methoxy-9H-carbazol-2-yloxy)ethylamine;
2-(7-benzyloxy-9H-carbazol-2-yloxy)ethylamine;
2-(7-trifluoromethyl-9H-carbazol-2-yloxy)ethylamine;
2-(7-methyl-9H-carbazol-2-yloxy)ethylamine;
2-(7-acetylamino-9H-carbazol-2-yloxy)ethylamine;
N-benzyl-2-(7-fluoro-9H-carbazol-2-yloxy)ethylamine;
N-benzyl-2-(7-chloro-9H-carbazol-2-yloxy)ethylamine;
N-benzyl-2-(7-bromo-9H-carbazol-2-yloxy)ethylamine;
N-benzyl-2-(7-methoxy-9H-carbazol-2-yloxy)ethylamine;
N-benzyl-2- (7-benzyloxy-9H-carbazol-2-yloxy)ethylamine;
N-benzyl-2-(7-trifluoromethyl-9H-carbazol-2-yloxy)ethylamine;
N-benzyl-2-(7-methyl-9H-carbazol-2-yloxy)ethylamine;
N-benzyl-2-(7-cyano-9H-carbazol-2-yloxy)ethylamine; and
N-benzyl-2-(7-acetylamino-9H-carbazol-2-yloxy)ethylamine;
or a salt thereof.

15. A compound of the general formula (IV): 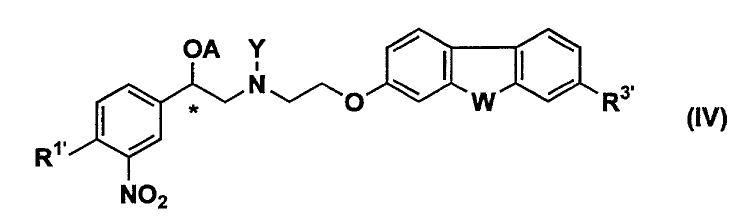 or a salt thereof,
wherein
A represents a hydrogen atom or a hydroxyl-protecting group;
R^{1'} represents a hydrogen atom, a halogen atom, or a protected hydroxyl group;
Y represents a hydrogen atom or an amine-protecting group;
R^{3'} represents OR', a halogen atom, a trifluoromethyl group, a lower alkyl group, a lower acyl group, NR⁴R^{4'}, a nitro group, or a cyano group;
R' represents a lower alkyl group, an optionally substituted lower acyl group, a benzyl group, or a hydroxyl-protecting group;
R⁴ and R^{4'} may be the same or different and represent a hydrogen atom, a lower alkyl group, a lower acyl group, a benzyl group, an amine-protecting group, or SO₂R⁵;
R⁵ represents a lower alkyl group or a benzyl group;
W represents an oxygen atom, a secondary nitrogen atom (NH), or a sulfur atom; and
* represents an asymmetric carbon atom.

16. A compound as claimed in claim 15 having the general formula (IV), wherein R^{3'} represents OR', a halogen atom, a trifluoromethyl group, a lower alkyl group, NR⁴R^{4'}, a nitro group, or a cyano group; R' represents a lower alkyl group, a benzyl group, or a hydroxyl-protecting group; R⁴ and R^{4'} may be the same or different and represent a hydrogen atom, a lower alkyl group, a benzyl group, or an amine-protecting group; W represents an oxygen atom, a secondary nitrogen atom (NH), or a sulfur atom; and * represents an asymmetric carbon atom, or a salt thereof.

17. A compound of the general formula (V): 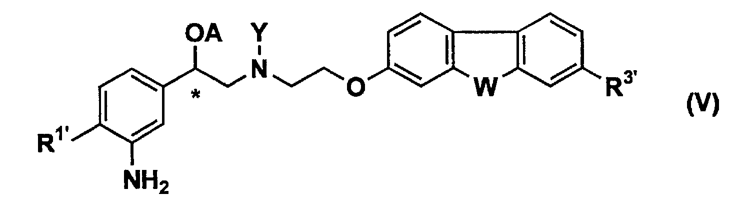 or a salt thereof,
wherein
A represents a hydrogen atom or a hydroxyl-protecting group;
R^{1'} represents a hydrogen atom, a halogen atom, or a protected hydroxyl group;
Y represents a hydrogen atom or an amine-protecting group;
R^{3'} represents OR', a halogen atom, a trifluoromethyl group, a lower alkyl group, a lower acyl group, NR⁴R^{4'}, a nitro group, or a cyano group;
R' represents a lower alkyl group, an optionally substituted lower acyl group, a benzyl group, or a hydroxyl-protecting group;
R⁴ and R^{4'} may be the same or different and represent a hydrogen atom, a lower alkyl group, a lower acyl group, a benzyl group, an amine-protecting group, or SO₂R⁵;
R⁵ represents a lower alkyl group or a benzyl group;
W represents an oxygen atom, a secondary nitrogen atom (NH), or a sulfur atom; and
* represents an asymmetric carbon atom.

18. A compound as claimed in claim 17 having the general formula (V), wherein R^{3'} represents OR', a halogen atom, a trifluoromethyl group, a lower alkyl group, NR⁴R^{4'}, a nitro group, or a cyano group; R' represents a lower alkyl group, a benzyl group, or a hydroxyl-protecting group; R⁴ and R^{4'} may be the same or different and represent a hydrogen atom, a lower alkyl group, a benzyl group, or an amine-protecting group; W represents an oxygen atom, a secondary nitrogen atom (NH), or a sulfur atom; and * represents an asymmetric carbon atom, or a salt thereof.

19. A compound of the general formula (VII): 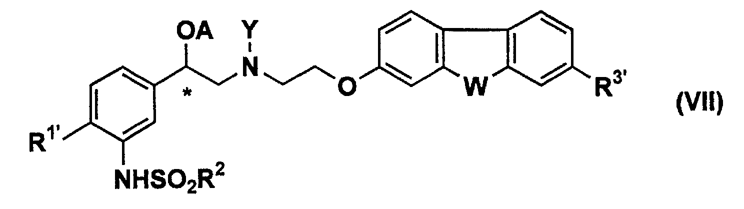 or a salt thereof,
wherein
A represents a hydrogen atom or a hydroxyl-protecting group;
R^{1'} represents a hydrogen atom, a halogen atom, or a protected hydroxyl group;
Y represents a hydrogen atom or an amine-protecting group;
R^{3'} represents OR', a halogen atom, a trifluoromethyl group, a lower alkyl group, a lower acyl group, NR⁴R^{4'}, a nitro group, or a cyano group;
R' represents a lower alkyl group, an optionally substituted lower acyl group, a benzyl group, or a hydroxyl-protecting group;
R⁴ and R^{4'} may be the same or different and represent a hydrogen atom, a lower alkyl group, a lower acyl group, a benzyl group, an amine-protecting group, or SO₂R⁵;
R⁵ represents a lower alkyl group or a benzyl group;
W represents an oxygen atom, a secondary nitrogen atom (NH), or a sulfur atom; and
* represents an asymmetric carbon atom.

20. A compound as claimed in claim 19 having the general formula (VII), wherein R^{3'} represents OR', a halogen atom, a trifluoromethyl group, a lower alkyl group, NR⁴R^{4'}, a nitro group, or a cyano group; R' represents a lower alkyl group, a benzyl group, or a hydroxyl-protecting group; R⁴ and R^{4'} may be the same or different and represent a hydrogen atom, a lower alkyl group, a benzyl group, or an amine-protecting group; W represents an oxygen atom, a secondary nitrogen atom (NH), or a sulfur atom; and * represents an asymmetric carbon atom, or a salt thereof.
